(19) Europäisches Patentamt
European Patent Office
Office européen des brevets

(11) EP 3 978 016 A1

(12) EUROPEAN PATENT APPLICATION
published in accordance with Art. 153(4) EPC

(43) Date of publication:
**06.04.2022 Bulletin 2022/14**

(21) Application number: **20812715.9**

(22) Date of filing: **28.05.2020**

(51) International Patent Classification (IPC):
***A61K 39/395*** *(2006.01)* ***A61P 1/04*** *(2006.01)*
***A61P 35/00*** *(2006.01)* ***C12Q 1/68*** *(2018.01)*
***C12Q 1/686*** *(2018.01)* ***C12Q 1/6869*** *(2018.01)*
***C12Q 1/6872*** *(2018.01)* ***C12Q 1/6886*** *(2018.01)*
***G01N 33/53*** *(2006.01)*

(52) Cooperative Patent Classification (CPC):
**C12Q 1/68; C12Q 1/686; C12Q 1/6869; C12Q 1/6872; C12Q 1/6886; G01N 33/53;**
A61K 39/395; A61P 1/04; A61P 35/00

(86) International application number:
**PCT/JP2020/021173**

(87) International publication number:
**WO 2020/241770 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2019 JP 2019103299**

(71) Applicants:
- **Tohoku University**
  **Sendai-shi, Miyagi 980-8577 (JP)**
- **Riken Genesis Co., Ltd.**
  **Tokyo 141-0032 (JP)**

(72) Inventors:
- **ISHIOKA, Chikashi**
  **Sendai-shi, Miyagi 980-8577 (JP)**
- **TAKAHASHI, Shin**
  **Sendai-shi, Miyagi 980-8577 (JP)**
- **OUCHI, Kouta**
  **Sendai-shi, Miyagi 980-8577 (JP)**
- **OKITA, Akira**
  **Sendai-shi, Miyagi 980-8577 (JP)**
- **SAITO, Tatsuro**
  **Tokyo 141-0032 (JP)**
- **NAKAMURA, Junko**
  **Tokyo 141-0032 (JP)**
- **TSUYADA, Akihiro**
  **Tokyo 141-0032 (JP)**

(74) Representative: **Hoffmann Eitle**
**Patent- und Rechtsanwälte PartmbB**
**Arabellastraße 30**
**81925 München (DE)**

# (54) METHOD FOR TESTING FOR SENSITIVITY OF CHEMOTHERAPY AGAINST COLORECTAL CANCER

(57) A method for testing for sensitivity of chemotherapy against colorectal cancer, the method using, as an indicator, methylation in at least one site selected from the group consisting of CpG sites comprised in regions (i) to (xvi) in DNA collected from a colorectal cancer patient (the regions (i) to (xvi) are as described herein).



Printed by Jouve, 75001 PARIS (FR)

## Description

Technical Field

[Cross-reference of related applications]

This application claims priority based on Japanese Patent Application No. 2019-103299, filed on May 31, 2019 (the entire disclosure of which is incorporated herein by reference). The present invention relates to a method for testing for sensitivity of chemotherapy against colorectal cancer.

Background Art

Colorectal cancer is a generic term for carcinomas occurring in the large intestine (colon, rectum, anus). Among cancers, colorectal cancer causes a large number of morbidity and mortality, and research on a treatment method thereof has been continued. In addition, as one of attempts of research on cancer therapy, "personalized medicine" has been studied in order to maximize therapeutic effect and minimize side effects as much as possible.

For example, Patent Literature 1 describes a method for predicting responsiveness to cancer chemotherapy using an anti-EGFR antibody of a colorectal cancer patient, using 24 genes represented by the following Target IDs: cg01791410, cg01802453, cg02484469, cg02916312, cg03839709, cg05218346, cg07005523, cg01068327, cg07258916, cg01360792, cg09767602, cg11092616, cg12646649, cg13261931,cg16041660,cg16958716,cgll188046,cg18412834,cg20012008, cg20265733, cg20339230, cg21787291, cg24792289, and cg27628784 as marker genes, and whether or not bases at specific positions of these marker genes are methylated as an indicator (Cited Literature 1, Example 8, and the like).

Citation List

Patent Literature

Patent Literature 1: WO 2016/060278 A1

Summary of Invention

Technical Problem

An object of the present invention is to provide a new method for testing for sensitivity of chemotherapy against colorectal cancer using, as an indicator, a methylation site that has not been conventionally known to be an indicator of responsiveness to chemotherapy of colorectal cancer.

Solution to Problem

Under such circumstances, the present inventors had intensively conducted studies, and resultantly found that sensitivity of chemotherapy against colorectal cancer can be efficiently tested, by using methylation in at least one site selected from the group consisting of CpG sites comprised in regions (i) to (xvi) described later, as an indicator. The present invention is based on such novel findings.

Accordingly, the present invention provides the following items:

Item 1. A method for testing for sensitivity of chemotherapy against colorectal cancer, the method using, as an indicator, methylation in at least one site selected from the group consisting of CpG sites comprised in the following regions (i) to (xvi) in DNA collected from a colorectal cancer patient:

Region (i): a region at positions 207307150 to 207309004 of chromosome 2
Region (ii): a region at positions 241758282 to 241760510 of chromosome 2
Region (iii): a region at positions 150802997 to 150805168 of chromosome 3
Region (iv): a region at positions 141347993 to 141348489 of chromosome 4
Region (v): a region at positions 186048714 to 186050048 of chromosome 4
Region (vi): a region at positions 17216679 to 17219240 of chromosome 5
Region (vii): a region at positions 37663982 to 37664539 of chromosome 6
Region (viii): a region at positions 39281183 to 39282332 of chromosome 6
Region (ix): a region at positions 42273390 to 42277951 of chromosome 7

Region (x): a region at positions 13039715 to 13043422 of chromosome 10
Region (xi): a region at positions 81664401 to 81665076 of chromosome 10
Region (xii): a region at positions 88126287 to 88127189 of chromosome 10
Region (xiii): a region at positions 61595807 to 61596627 of chromosome 11
Region (xiv): a region at positions 23820337 to 23822266 of chromosome 14
Region (xv): a region at positions 44895317 to 44896749 of chromosome 17
Region (xvi): a region at positions 39520228 to 39523159 of chromosome 19.

Item 2. The method according to item 1, comprising detecting methylation in at least one site selected from the group consisting of bases at:

positions 207307150, 207307490, 207307544, 207307622, 207307732, 207308004, 207308087, 207308244, 207308375, 207308829, 207309003, 241758282, 241758399, 241758805, 241758901, 241759279, 241759414, 241760025, 241760164, 241760190, and 241760509 of chromosome 2,
positions 150802997, 150803295, 150803307, 150803666, 150803669, 150804058, 150804063, 150804313, 150804490, 150804696, 150804719, and 150805167 of chromosome 3,
positions 141347993, 141348043, 141348167, 141348307, 141348488, 186048714, 186048907, 186049687, 186049926, and 186050047 of chromosome 4, positions 17216679, 17216922, 17217093, 17217877, 17218089, 17218278, 17218308, 17218547, 17218778, 17219020, 17219128, 17219226, and 17219239 of chromosome 5,
positions 37663982, 37664451, 37664538, 39281183, 39281421, 39281450, 39281541, 39281694, 39281885, 39282164, 39282316, and 39282331 of chromosome 6,
positions 42273390, 42275601, 42275813, 42275870, 42275872, 42276004, 42276814, 42276816, 42276819, 42276848, 42276881, 42276890, 42276941, 42276981, 42277044, 42277066, 42277071, 42277347, 42277375, 42277394, 42277410, 42277807, and 42277950 of chromosome 7,
positions 13039715, 13041989, 13043313, 13043421, 81664401, 81664567, 81664583, 81664698, 81664955, 81665075, 88126287, 88126291, 88126299, 88126306, 88126853, and 88127188 of chromosome 10,
positions 61595807, 61595956, 61596068, 61596307, 61596333, 61596405, and 61596626 of chromosome 11,
positions 23820337, 23821149, 23821229, 23821435, 23821445, 23821570, 23821596, 23821902, 23822017, and 23822265 of chromosome 14,
positions 44895317, 44896017, 44896080, 44896147, 44896162, 44896166, 44896168, 44896212, 44896223, 44896424, and 44896748 of chromosome 17, and
positions 39520228, 39521931, 39522418, 39522548, 39522747, 39522944, 39523083, and 39523158 of chromosome 19, and using the detection result as an indicator of sensitivity of chemotherapy against colorectal cancer.

Item 3. The method according to item 1 or 2, in which methylation in at least one site selected from the group consisting of CpG sites comprised in the following regions (i') to (iv'), regions (viii') to (x'), and regions (xii') to (xvi') is used as an indicator:

Region (i'): a region at positions 207307490 to 207308376 of chromosome 2
Region (ii') a region at positions 241758805 to 241760510 of chromosome 2
Region (iii'): a region at positions 150802997 to 150804720 of chromosome 3
Region (iv'): a region at positions 141348167 to 141348308 of chromosome 4
Region (viii'): a region at positions 39281541 to 39282165 of chromosome 6
Region (ix'): a region at positions 42275870 to 42276817 of chromosome 7
Region (x'): a region at positions 13041989 to 13043422 of chromosome 10
Region (xii'): a region at positions 88126287 to 88126307 of chromosome 10
Region (xiii'): a region at positions 61595807 to 61596334 of chromosome 11
Region (xiv'): a region at positions 23821596 to 23822266 of chromosome 14
Region (xv'): a region at positions 44895317 to 44896425 of chromosome 17
Region (xvi'): a region at positions 39520228 to 39523084 of chromosome 19.

Item 4. The method according to item 1, comprising detecting methylation in at least one site selected from the group consisting of bases at:

positions 207308141, 207308149, 207308153, 207308172, 207308177, 207308181, 207308185, 207308244, 207308247, 207308267, 241758885, 241758889, 241758896, 241758901, 241758919, 241758922, 241758931, 241758936, 241758940, 241759003, 241759005, and 241759009 of chromosome 2,
positions 150804417, 150804420, 150804474, 150804479, 150804486, 150804490, 150804496, 150804504, and 150804507 of chromosome 3,
positions 141348224, 141348232, 141348272, 141348282, 141348289, 141348307, 141348318, 141348324, 186049623, 186049636, 186049659, 186049670, 186049685, 186049687, 186049689, and 186049705 of chromosome 4,
positions 17216904, 17216916, 17216922, 17216940, 17216949, 17216952, 17217003, 17217011, and 17217017 of chromosome 5,
positions 37664352, 37664366, 37664419, 37664424, 37664451, 37664460, 39281692, 39281694, 39281723, 39281729, 39281735, 39281738, 39281797, 39281806, 39281808, and 39281813 of chromosome 6,
positions 42276764, 42276767, 42276769, 42276783, 42276810, 42276814, 42276816, 42276819, 42276825, 42276852, 42276862, and 42276874 of chromosome 7,
positions 13043231, 13043247, 13043285, 13043288, 13043313, 13043321, 13043333, 81664567, 81664573, 81664583, 81664598, 81664600, 81664606, 81664614, 81664636, 81664656, 88126243, 88126250, 88126259, 88126285, 88126287, 88126291, 88126299, 88126306, and 88126310 of chromosome 10,
positions 61595796, 61595807, 61595815, 61595818, 61595820, 61595829, 61595843, and 61595852 of chromosome 11,
positions 23822015, 23822017, 23822043, 23822054, 23822073, 23822075, and 23822079 of chromosome 14,
positions 44896314, 44896316, 44896324, 44896386, 44896390, 44896401, 44896424, 44896443, and 44896450 of chromosome 17, and
positions 39522493, 39522510, 39522533, 39522537, 39522548, 39522550, 39522552, 39522582, 39522587, 39522591, and 39522608 of chromosome 19, and using the detection result as an indicator of sensitivity of chemotherapy against colorectal cancer.

Item 5: The method according to any one of items 1 to 4, in which methylation in at least one site selected from the group consisting of CpG sites comprised in the following regions is used as an indicator:

a region at positions 207308134 to 207308268 of chromosome 2
a region at positions 241758885 to 241759011 of chromosome 2
a region at positions 150804402 to 150804508 of chromosome 3
a region at positions 141348215 to 141348329 of chromosome 4
a region at positions 186049616 to 186049706 of chromosome 4
a region at positions 17216901 to 17217025 of chromosome 5
a region at positions 37664344 to 37664472 of chromosome 6
a region at positions 39281671 to 39281814 of chromosome 6
a region at positions 42276764 to 42276875 of chromosome 7
a region at positions 13043227 to 13043334 of chromosome 10
a region at positions 81664566 to 81664662 of chromosome 10
a region at positions 88126243 to 88126334 of chromosome 10
a region at positions 61595787 to 61595864 of chromosome 11
a region at positions 23821996 to 23822092 of chromosome 14
a region at positions 44896309 to 44896451 of chromosome 17
a region at positions 39522493 to 39522609 of chromosome 19.

Item 6. The method according to any one of items 1 to 5, in which the CpG site is located in a region to which an oligonucleotide of at least one sequence selected from the group consisting of SEQ ID NOs: 1 to 48 hybridizes, in DNA obtained by bisulfite treatment of DNA containing the CpG site that is methylated.

Item 7. The method according to any one of items 1 to 6, comprising the steps of:

(1) detecting a plurality of CpG sites comprised in each of at least two regions among the regions (i) to (xvi) and measuring frequency of methylation of the detected CpG sites,
(2) in said each region detected in the step (1), when the frequency of methylation in the region was equal to or more than a preset value, determining the region as a highly methylated region, and
(3) in said each region measured in the step (1), when a ratio of the regions determined as the high methylated region in the step (2) is equal to or more than a preset ratio, determining that there is no sensitivity of chemotherapy against colorectal cancer.

Item 8. The method according to any one of items 1 to 6, comprising the steps of:

(1) detecting a plurality of CpG sites comprised in each of at least two regions among the regions (i) to (xvi) and measuring frequency of methylation of the detected CpG sites,
(2) in said each region measured in the step (1), when the frequency of methylation in the region was equal to or more than a preset value, determining the region as a highly methylated region, and
(3) in said each region measured in the step (1), when a ratio of the regions determined as the high methylated region in the step (2) is less than a preset ratio, determining that there is sensitivity of chemotherapy against colorectal cancer.

Item 9. The method according to item 7 or 8, in which the frequency of methylation in each region in the step (1) is measured by real-time PCR, and when a ΔCt value that is a difference between a Ct value measured by real-time PCR and a Ct value of a control reaction is less than a preset value, the region is defined as the highly methylated region.
Item 10. The method according to any one of items 7 to 9, in which the frequency of methylation is measured for at least 8 regions among the regions (i) to (xvi) in the step (1).
Item 11. The method according to any one of items 1 to 10, in which the DNA collected from the colorectal cancer patient is DNA prepared from colorectal tissue, blood, serum, plasma, feces, intestinal lavage solution or enema lavage solution collected from the colorectal cancer patient.
Item 12. The method according to any one of items 1 to 11, in which the chemotherapy against colorectal cancer is a chemotherapy using an anti-EGFR antibody.
Item 13. The method according to item 12, in which the anti-EGFR antibody is at least one selected from the group consisting of cetuximab and panitumumab.
Item 14. A kit for acquiring information that can be an indicator of presence or absence of sensitivity of chemotherapy against colorectal cancer, the kit comprising a primer set for analyzing methylation in at least one site selected from the group consisting of CpG sites comprised in the following regions (i) to (xvi) in DNA collected from a colorectal cancer patient:

Region (i): a region at positions 207307150 to 207309004 of chromosome 2
Region (ii): a region at positions 241758282 to 241760510 of chromosome 2
Region (iii): a region at positions 150802997 to 150805168 of chromosome 3
Region (iv): a region at positions 141347993 to 141348489 of chromosome 4
Region (v): a region at positions 186048714 to 186050048 of chromosome 4
Region (vi): a region at positions 17216679 to 17219240 of chromosome 5
Region (vii): a region at positions 37663982 to 37664539 of chromosome 6
Region (viii): a region at positions 39281183 to 39282332 of chromosome 6
Region (ix): a region at positions 42273390 to 42277951 of chromosome 7
Region (x): a region at positions 13039715 to 13043422 of chromosome 10
Region (xi): a region at positions 81664401 to 81665076 of chromosome 10
Region (xii): a region at positions 88126287 to 88127189 of chromosome 10
Region (xiii): a region at positions 61595807 to 61596627 of chromosome 11
Region (xiv): a region at positions 23820337 to 23822266 of chromosome 14
Region (xv): a region at positions 44895317 to 44896749 of chromosome 17
Region (xvi): a region at positions 39520228 to 39523159 of chromosome 19.

Item 15. The kit according to item 14, in which the primer set is a primer set for analyzing methylation of a CpG site by at least one method selected from the group consisting of a methylation-specific PCR method, a sequencing method, and a mass spectrometry method.
Item 16. The kit according to item 14 or 15, in which the primer set comprises a primer set capable of amplifying at least one of the following regions after bisulfite treatment when a CpG site comprised in the region is methylated:

a region comprising positions 207308134 to 207308267 of chromosome 2
a region comprising positions 241758885 to 241759011 of chromosome 2
a region comprising positions 150804402 to 150804508 of chromosome 3
a region comprising positions 141348215 to 141348329 of chromosome 4
a region comprising positions 186049616 to 186049705 of chromosome 4
a region comprising positions 17216901 to 17217025 of chromosome 5
a region comprising positions 37664344 to 37664472 of chromosome 6
a region comprising positions 39281671 to 39281814 of chromosome 6
a region comprising positions 42276765 to 42276874 of chromosome 7
a region comprising positions 13043227 to 13043334 of chromosome 10
a region comprising positions 81664566 to 81664662 of chromosome 10

a region comprising positions 88126244 to 88126334 of chromosome 10
a region comprising positions 61595787 to 61595864 of chromosome 11
a region comprising positions 23821996 to 23822092 of chromosome 14
a region comprising positions 44896309 to 44896450 of chromosome 17
a region comprising positions 39522494 to 39522608 of chromosome 19.

Item 17. A use of a primer set for analyzing methylation in at least one site selected from the group consisting of CpG sites comprised in the following regions (i) to (xvi) in DNA collected from a colorectal cancer patient, in order to produce a kit for acquiring information that can be an indicator of presence or absence of sensitivity of chemotherapy against colorectal cancer:

Region (i): a region at positions 207307150 to 207309004 of chromosome 2
Region (ii): a region at positions 241758282 to 241760510 of chromosome 2
Region (iii): a region at positions 150802997 to 150805168 of chromosome 3
Region (iv): a region at positions 141347993 to 141348489 of chromosome 4
Region (v): a region at positions 186048714 to 186050048 of chromosome 4
Region (vi): a region at positions 17216679 to 17219240 of chromosome 5
Region (vii): a region at positions 37663982 to 37664539 of chromosome 6
Region (viii): a region at positions 39281183 to 39282332 of chromosome 6
Region (ix): a region at positions 42273390 to 42277951 of chromosome 7
Region (x): a region at positions 13039715 to 13043422 of chromosome 10
Region (xi): a region at positions 81664401 to 81665076 of chromosome 10
Region (xii): a region at positions 88126287 to 88127189 of chromosome 10
Region (xiii): a region at positions 61595807 to 61596627 of chromosome 11
Region (xiv): a region at positions 23820337 to 23822266 of chromosome 14
Region (xv): a region at positions 44895317 to 44896749 of chromosome 17
Region (xvi): a region at positions 39520228 to 39523159 of chromosome 19.

Item 18. The use according to item 17, in which the primer set is a primer set for analyzing methylation of a CpG site by at least one method selected from the group consisting of a methylation-specific PCR method, a sequencing method, and a mass spectrometry method.

Item 19. The use according to Item 17 or 18, in which the primer set comprises a primer set capable of amplifying at least one of the following regions after bisulfite treatment when a CpG site comprised in the region is methylated:

a region comprising positions 207308134 to 207308267 of chromosome 2
a region comprising positions 241758885 to 241759011 of chromosome 2
a region comprising positions 150804402 to 150804508 of chromosome 3
a region comprising positions 141348215 to 141348329 of chromosome 4
a region comprising positions 186049616 to 186049705 of chromosome 4
a region comprising positions 17216901 to 17217025 of chromosome 5
a region comprising positions 37664344 to 37664472 of chromosome 6
a region comprising positions 39281671 to 39281814 of chromosome 6
a region comprising positions 42276765 to 42276874 of chromosome 7
a region comprising positions 13043227 to 13043334 of chromosome 10
a region comprising positions 81664566 to 81664662 of chromosome 10
a region comprising positions 88126244 to 88126334 of chromosome 10
a region comprising positions 61595787 to 61595864 of chromosome 11
a region comprising positions 23821996 to 23822092 of chromosome 14
a region comprising positions 44896309 to 44896450 of chromosome 17
a region comprising positions 39522494 to 39522608 of chromosome 19.

Advantageous Effects of Invention

According to the present invention, it is possible to provide a new method for testing for sensitivity of chemotherapy against colorectal cancer using, as an indicator, a methylation site that has not been conventionally known to be an indicator of responsiveness to chemotherapy of colorectal cancer.

Brief Description of Drawings

Fig. 1 is a graph summarizing progression-free survival: PFS for each group in Example 2.
Fig. 2 is a graph summarizing progression-free survival: PFS for each group in Example 3.
Fig. 3 is a graph summarizing progression-free survival: PFS for each group in 83 cases of advanced and recurrent colorectal cancer in which KRAS gene in Example 4 is wild type.
Fig. 4 is a graph summarizing progression-free survival: PFS for each group in 65 cases in which RAS gene in Example 4 is wild type.
Fig. 5 shows DNA methylation analysis results around Target ID cg07319626 in Examples.
Fig. 6 shows DNA methylation analysis results around Target ID cg02610058 in Examples.
Fig. 7 shows DNA methylation analysis results around Target ID cg13803214 in Examples.
Fig. 8 shows DNA methylation analysis results around Target ID cg14235416 in Examples.
Fig. 9 shows DNA methylation analysis results around Target ID cg24642320 in Examples.
Fig. 10 shows DNA methylation analysis results around Target ID cg25203704 in Examples.
Fig. 11 shows DNA methylation analysis results around Target ID cg26129310 in Examples.
Fig. 12 shows DNA methylation analysis results around Target ID cg18960642 in Examples.
Fig. 13 shows DNA methylation analysis results around Target ID cg07413609 in Example.
Fig. 14 shows DNA methylation analysis results around Target ID cg22738219 in Examples.
Fig. 15 shows DNA methylation analysis results around Target ID cg20629468 in Examples.
Fig. 16 shows DNA methylation analysis results around Target ID cg20649951 in Examples.
Fig. 17 shows DNA methylation analysis results around Target ID cg25303599 in Examples.
Fig. 18 shows DNA methylation analysis results around Target ID cg01557297 in Examples.
Fig. 19 shows DNA methylation analysis results around Target ID cg12379948 in Examples.
Fig. 20 shows DNA methylation analysis results around Target ID cg14730085 in Examples.

Description of Embodiments

(1. Method for testing for sensitivity of chemotherapy against colorectal cancer)

The present invention provides a method for testing for sensitivity of chemotherapy against colorectal cancer, using methylation in at least one site selected from the group consisting of CpG sites comprised in the following regions (i) to (xvi) in DNA collected from a colorectal cancer patient as an indicator:

Region (i): a region at positions 207307150 to 207309004 (preferably, positions 207307490 to 207308376) of chromosome 2
Region (ii): a region at positions 241758282 to 241760510 (preferably, positions 241758805 to 241760510) of chromosome 2
Region (iii): a region at positions 150802997 to 150805168 (preferably, positions 150802997 to 150804720) of chromosome 3
Region (iv): a region at positions 141347993 to 141348489 (preferably, positions 141348167 to 141348308) of chromosome 4
Region (v): a region at positions 186048714 to 186050048 of chromosome 4
Region (vi): a region at positions 17216679 to 17219240 of chromosome 5
Region (vii): a region at positions 37663982 to 37664539 of chromosome 6
Region (viii): a region at positions 39281183 to 39282332 (preferably, positions 39281541 to 39282165) of chromosome 6
Region (ix): a region at positions 42273390 to 42277951 (preferably, positions 42275870 to 42276817) of chromosome 7
Region (x): a region at positions 13039715 to 13043422 (preferably, positions 13041989 to 13043422) of chromosome 10
Region (xi): a region at positions 81664401 to 81665076 of chromosome 10
Region (xii): a region at positions 88126287 to 88127189 (preferably, positions 88126287 to 88126307) of chromosome 10
Region (xiii): a region at positions 61595807 to 61596627 (preferably, positions 61595807 to 61596334) of chromosome 11
Region (xiv): a region at positions 23820337 to 23822266 (preferably, positions 23821596 to 23822266) of chromosome 14
Region (xv): a region at positions 44895317 to 44896749 (preferably, positions 44895317 to 44896425) of chromosome 17
Region (xvi): a region at positions 39520228 to 39523159 (preferably, positions 39520228 to 39523084) of chromosome 19

The test method of the present invention is performed in vitro.

(1.1. Explanation of terms)

In the present invention, DNA collected from a colorectal cancer patient is to be measured. In the present invention, colorectal cancer is a carcinoma occurring in the large intestine (colon (including cecum) and rectum), and also includes a carcinoma occurring in the anal canal. The subject of the present invention is a human. In the present invention, the colorectal cancer patient includes not only a human suffering from colorectal cancer but also humans suspected of suffering from colorectal cancer and in need of investigation of cancer chemotherapy responsiveness. Moreover, in the present invention, the colorectal cancer patient also includes a human from which colorectal cancer has already been removed by surgical operation.

In the present invention, DNA is extracted from a sample collected from the colorectal cancer patient. The sample from which DNA is extracted is not particularly limited, and examples thereof include colorectal tissue, blood, serum, plasma, feces, intestinal lavage solution, enema lavage solution and the like collected from the colorectal cancer patient. A method for extracting DNA from these samples is also not particularly limited, and the extraction can be performed using a known method or based on a known method. For example, it can be performed using a commercially available kit such as EZ DNA Methylation-GOLD QIAampKit (ZYMO RESEARCH), QIAamp DNA Micro Kit (QIAGEN), or NucleoSpinR Tissue (TAKARA).

In the present invention, the chemotherapy against colorectal cancer is not particularly limited, and examples thereof include chemotherapy, a therapeutic method using a molecular target drug, combinations thereof, and the like. The drug used for chemotherapy is not particularly limited, and examples thereof include oxaliplatin, irinotecan, fluorouracil, trifluridine/tipiracil hydrochloride, and the like. The molecular target drug is also not particularly limited, and examples thereof include an anti-EGFR antibody, an anti-VEGF antibody, an oral multi-kinase inhibitor, and the like.

In the present invention, the "anti-EGFR antibody" refers to an antibody specific to EGFR (epidermal growth factor receptor) or an immunologically active fragment thereof. The anti-EGFR antibody is not particularly limited, and examples thereof include cetuximab, panitumumab, and the like.

In the present invention, the sensitivity of chemotherapy against colorectal cancer means the sensitivity of the patient to cancer chemotherapy as described above, and a case where the cancer chemotherapy is effective is expressed as sensitivity, and a case where the cancer chemotherapy is not effective is expressed as resistance.

DNA methylation can usually occur at a carbon atom at position 5 of a pyrimidine ring of cytosine or a nitrogen atom at position 6 of a purine ring of adenine, which constitutes DNA. In somatic tissues of adult mammals, DNA methylation is typically likely to occur at CpG sites (dinucleotide sites where cytosine and guanine are adjacent). In the present invention, when each region on the DNA is referred to as "a region at positions Y to Z of chromosome X", the reference sequence is "hg19". When the base sequence of DNA in a sample of a cancer patient to be tested includes addition, substitution and/or deletion of a base sequence with respect to a base sequence recorded in the reference sequence, positions of TargetID and the base mean positions corresponding to positions in a human genome sequence recorded in the "hg19" in consideration of the addition, substitution and/or deletion. In addition, in the present invention, "detection of methylation of a base at position Y of chromosome X" includes not only a case where the base at position Y of chromosome X is cytosine and methylation of the cytosine is detected, but also a case where the base at position Y of chromosome X is guanine and methylation of the corresponding cytosine in the complementary strand is detected. In the present invention, "detection of methylation" means to confirm the presence or absence of methylation of cytosine present at a certain position unless otherwise indicated.

(1.2. Methylation detection method and method of measuring frequency of methylation)

The method of detecting methylation of the CpG site is not limited, and examples thereof include a method of calculating a $\beta$ value using a bead array described later, a method of obtaining a $\Delta$Ct value using real-time PCR, and the like. Also, in the present invention, the frequency of methylation can be used as an indicator of methylation. The method for measuring the frequency of methylation of CpG sites comprised in a predetermined region is not limited in the present invention, and examples thereof include a method in which a $\beta$ value is calculated using the bead array described later to detect methylation at a predetermined position in the region, and the frequency of methylation is calculated from the detection result, a method in which the frequency is measured by real-time PCR using a $\Delta$Ct value

as an indicator of the frequency of methylation, and the like, and the method using real-time PCR and the like are preferable.

When methylation of the CpG site is detected by calculating the β value using a bead array, methylation can be detected using a method using, for example, a bead array of Illumina, Inc. (Infinium (registered trademark) Human-Methylation 450 BeadChip or Infinium MethylationEPIC BeadChip). In this method, unmethylated cytosine (non-methylated cytosine) in DNA is converted to uracil by bisulfite treatment, whereby methylated cytosine is distinguished from non-methylated cytosine. Then, after hybridization of probes immobilized on two beads, a methylation probe (M type) and a non-methylation probe (U type), specific to each site, a single base extension reaction using a labeled ddNTP is performed, and the ratio of methylation to non-methylation is calculated from the fluorescence intensity signal. This makes it possible to easily perform comprehensive DNA methylation analysis. When such a method is used, the frequency of methylation can be measured using, for example, a β value. Specifically, the β value is an indicator of the ratio of methylation (methylation/methylation + non-methylation), and is calculated by the following equation.

$$\beta \text{ Value} = (\text{Maximum value of fluorescence value of methyl detection probe})/(\text{Maximum value of fluorescence value of probe for non-methyl detection} + \text{Maximum value of fluorescence value of probe for methyl detection} + 100).$$

In the present invention, the reference value of the β value can be appropriately set, and for example, it is preferable to set as highly methylated when the β value is more than 0.4, and more preferable to set as highly methylated when the β value is more than 0.3.

Also, in another preferred embodiment of the present invention, real-time PCR can be used. Specifically, real-time PCR (methylation-specific PCR) is performed using a forward primer, a reverse primer, and/or a probe specific to a methylated DNA sequence. Cytosine in non-methylated DNA is converted into uracil by bisulfite treatment, and a sequence of the non-methylated DNA is different from those of the forward primer and reverse primer specific to a methylated DNA sequence. Therefore, PCR amplification does not occur, and only methylated DNA is amplified by PCR.

A probe is used to detect a PCR amplification product. In a typical embodiment, a probe labeled with a fluorescent substance and a quenching substance at both ends can be used. Generation of fluorescence is suppressed by the quenching substance before the reaction, but when the probe is degraded by activity of DNA polymerase during the PCR reaction, the fluorescent substance is released to emit fluorescence. A fluorescence signal generated by degradation of the probe is detected, and a Cycle threshold (Ct) value, which is the number of cycles when the fluorescence intensity reaches a certain amount, is calculated. In methylation-specific PCR, a probe hybridizes to a methylated DNA sequence, and then is degraded and emits light. The Ct value can be obtained by detecting the fluorescence signal. On the other hand, since the probe does not hybridize to the non-methylated DNA sequence, a fluorescence signal due to degradation of the probe is not detected, and the Ct value becomes very large or cannot be obtained. Normally, since the Ct value is also affected by the amount and quality of specimen DNA, real-time PCR of a control region is simultaneously performed in the same reaction in order to correct the effect. In that case, preferably, the control primer and probe are designed so that a region different from a region to be detected for DNA methylation can be amplified and detected. Further preferably, the control primer is designed so as not to include a CpG site in the region to be amplified. Therefore, the control region can be amplified regardless of the presence or absence of methylation. Then, difference between the Ct value of the control reaction and the Ct value of the methylation detection reaction is calculated as a ΔCt value, and a result can be determined according to the ΔCt value. The ΔCt value increases in the non-methylated DNA, and the ΔCt value decreases in the methylated DNA.

In the present embodiment, examples of the measurement of the frequency of methylation in each of the regions (i) to (xvi) include the following method. First, when all CpG sites to be detected are methylated, a range including the CpG sites is amplified by PCR after bisulfite treatment, and primers and probe sets are designed so that the CpG sites can be detected. Further, a control region is also set to perform real-time PCR to calculate a ΔCt value. When ΔCt is small, the frequency of methylation can be considered as high, and when ΔCt is large, the frequency of methylation can be considered as low. Therefore, when ΔCt is less than predetermined value Tb, the region can be set as a highly methylated region. In addition, the ΔCt value is calculated by the above method, and when ΔCt is equal to or more than the predetermined value Tb, the region can be set as a low methylated region. In the present invention, the reference value Tb of the ΔCt value can be appropriately set, and can be appropriately set in the range of, for example, $3 \leq Tb \leq 7$, preferably $3.5 \leq Tb \leq 5$, further preferably $3.5 \leq Tb \leq 4.5$, and particularly preferably $Tb = 4$. More specifically, for example, it is preferable to set as highly methylated when the ΔCt value is 6 or less, and more preferable to set as highly methylated when the ΔCt value is less than 4. In the case of using real-time PCR, base length of region amplified by a primer set can be appropriately set, and can be set in the range of, for example, 75 to 200 bases, preferably 90 to 160 bases, and more preferably 100 to 150 bases. The number of CpG sites comprised in the region to be amplified can be one site or plural sites (for example, 1 or more sites, preferably 3 or more sites, more preferably 5 or more sites, more preferably 8 or more sites, more preferably 10 or more sites, more preferably 13 or more sites, and further preferably 15 or more sites). A method of setting the region to be amplified is not particularly limited, and for example, the region can be set so as to comprise one site or plural sites (for example, 1 or more sites, preferably 3 or more sites, more preferably 5 or more sites, more preferably 8 or more sites, more preferably 10 or more sites, more preferably 13 or more sites, and further preferably 15 or more sites) selected from the group consisting of (i-1) to (i-11), (ii-1) to (ii-10), (iii-1) to (iii-12), (iv-1) to (iv-5), (v-1) to (v-5), (vi-1) to (vi-13), (vii-1) to (vii-3), (viii-1) to (viii-9), (ix-1) to (ix-23), (x-1) to (x-4), (xi-1) to (xi-6), (xii-1) to (xii-6), (xiii-1) to (xiii-7), (xiv-1) to (xiv-10), (xv-1) to (xv-11), and (xvi-1) to (xvi-8) described later.

In another exemplary embodiment, the method of the present invention includes a method in which the frequency of methylation in at least one site selected from the group consisting of CPG sites comprised in the regions (i) to (xvi) is used as an indicator. In this embodiment, the method of the present invention comprises a step of measuring the frequency of methylation in at least one site selected from the group consisting of CPG sites comprised in the regions (i) to (xvi). The method for measuring the frequency of methylation of each of the regions (i) to (xvi) is not particularly limited, and examples thereof include a method in which a CPG site at a predetermined position in each region is set as a representative CPG site of the region, and the methylation thereof is detected. Here, the "frequency of methylation" is an indicator indicating a ratio of methylated (or unmethylated) cytosine. For example, it can be expressed as a percentage. In the present invention, the frequency of methylation in the CPG site can be rephrased as frequency of methylation in bases constituting the CPG site. In one embodiment, it is calculated by dividing the number of cytosines contained in the CPG site to be detected in the region (including cytosines in the complementary strands corresponding to glycines in the CPG site of the reference sequence) by the number of methylated cytosines (or the number of unmethylated cytosines). Even when the number of CPG sites to be detected in the region is one, the frequency of methylation can be calculated. In this case, the frequency of methylation is indicated at any of 0%, 50%, and 100%.

In this embodiment, when the ratio of the number of regions determined to be highly methylated regions to the number of regions to be measured in the regions (i) to (xvi) is equal to or more than reference value Tc, it can be determined that there is no sensitivity of chemotherapy against colorectal cancer. For example, the reference value Tc can be appropriately set in the range of $0.2 \leq Tc$, preferably $0.2 \leq Tc \leq 0.8$, more preferably $0.3 \leq Tc \leq 0.7$, and further preferably $0.4 \leq Tc \leq 0.6$. More specifically, it can be set, for example, Tc = 0.5.

In addition, in another embodiment of the present invention, for example, when the frequency of methylation of the region (i) is measured, the frequency of methylation of the region (i) can be measured by detecting methylation of a representative CpG site, instead of detecting methylation of all CpG sites comprised in the region. For example, the frequency of methylation of the region (i) can be measured by detecting methylation of base(s) in at least one site (preferably 2 or more sites, more preferably 6 or more sites, more preferably 8 or more sites, and further preferably 11 sites) selected from the group consisting of the following (i-1) to (i-11):

(i-1) a base at position 207307150 of chromosome 2
(i-2) a base at position 207307490 of chromosome 2
(i-3) a base at position 207307544 of chromosome 2
(i-4) a base at position 207307622 of chromosome 2
(i-5) a base at position 207307732 of chromosome 2
(i-6) a base at position 207308004 of chromosome 2
(i-7) a base at position 207308087 of chromosome 2
(i-8) a base at position 207308244 of chromosome 2
(i-9) a base at position 207308375 of chromosome 2
(i-10) a base at position 207308829 of chromosome 2
(i-11) a base at position 207309003 of chromosome 2.

In particular, it is preferable to detect methylation of at least (i-8) among the positions of the bases. The method of detecting methylation is not limited, and examples thereof include a method of calculating a $\beta$ value using a bead array described later, a method of calculating a $\Delta$Ct value using real-time PCR, and the like, and the method of calculating a $\beta$ value using a bead array is preferable. In the present embodiment, when methylation at one site among (i-1) to (i-11) is detected, considering a position where the detection is performed as a representative position in the region (i), the region (i) can be set as a highly methylated region when a base at the position is methylated (for example, a case where the $\beta$ value is more than or equal to a predetermined value, a case where the $\Delta$Ct value is less than or equal to a predetermined value), and otherwise, the region (i) can be set as a low methylated region. Further, in the present embodiment, when methylation at two or more sites among (i-1) to (i-11) is detected, considering the position as a representative position in the region (i), the region (i) can be set as a highly methylated region when bases of more than or equal to a predetermined ratio (for example, half or more, 60% or more, or 70% or more) of the bases at the positions are methylated (for example, a case where the β value is more than or equal to a predetermined value, a case where the ΔCt value is less than or equal to a predetermined value), and otherwise, the region (i) can be set as a low methylated region.

In addition, in one embodiment of the present invention, for example, when the frequency of methylation of the region (ii) is measured, the frequency of methylation of the region (ii) can be measured by detecting methylation of base(s) in at least one site (preferably 2 or more sites, more preferably 6 or more sites, more preferably 7 or more sites, and further preferably 10 sites) selected from the group consisting of the following (ii-1) to (ii-10):

(ii-1) a base at position 241758282 of chromosome 2
(ii-2) a base at position 241758399 of chromosome 2
(ii-3) a base at position 241758805 of chromosome 2
(ii-4) a base at position 241758901 of chromosome 2
(ii-5) a base at position 241759279 of chromosome 2
(ii-6) a base at position 241759414 of chromosome 2
(ii-7) a base at position 241760025 of chromosome 2
(ii-8) a base at position 241760164 of chromosome 2
(ii-9) a base at position 241760190 of chromosome 2
(ii-10) a base at position 241760509 of chromosome 2.

In particular, it is preferable to detect methylation of at least (ii-4) among the positions of the bases. In such an embodiment, the method of detecting methylation, and in which case the region (ii) is set as a highly methylated region and in which case the region (ii) is set as a low methylated region are the same as those described above for the region (i).

In addition, in one embodiment of the present invention, for example, when the frequency of methylation of the region (iii) is measured, the frequency of methylation of the region (iii) can be measured by detecting methylation of base(s) in at least one site (preferably 2 or more sites, more preferably 6 or more sites, more preferably 8 or more sites, and further preferably 12 sites) selected from the group consisting of the following (iii-1) to (iii-12):

(iii-1) a base at position 150802997 of chromosome 3
(iii-2) a base at position 150803295 of chromosome 3
(iii-3) a base at position 150803307 of chromosome 3
(iii-4) a base at position 150803666 of chromosome 3
(iii-5) a base at position 150803669 of chromosome 3
(iii-6) a base at position 150804058 of chromosome 3
(iii-7) a base at position 150804063 of chromosome 3
(iii-8) a base at position 150804313 of chromosome 3
(iii-9) a base at position 150804490 of chromosome 3
(iii-10) a base at position 150804696 of chromosome 3
(iii-11) a base at position 150804719 of chromosome 3
(iii-12) a base at position 150805167 of chromosome 3.

In particular, it is preferable to detect methylation of at least (iii-9) among the positions of the bases. In such an embodiment, the method of detecting methylation, and in which case the region (iii) is set as a highly methylated region and in which case the region (iii) is set as a low methylated region are the same as those described above for the region (i).

In addition, in one embodiment of the present invention, for example, when the frequency of methylation of the region (iv) is measured, the frequency of methylation of the region (iv) can be measured by detecting methylation of base(s) in at least one site (preferably 2 or more sites, more preferably 3 or more sites, more preferably 4 or more sites, and further preferably 5 sites) selected from the group consisting of the following (iv-1) to (iv-5):

(iv-1) a base at position 141347993 of chromosome 4
(iv-2) a base at position 141348043 of chromosome 4
(iv-3) a base at position 141348167 of chromosome 4
(iv-4) a base at position 141348307 of chromosome 4
(iv-5) a base at position 141348488 of chromosome 4.

In particular, it is preferable to detect methylation of at least (iv-4) among the positions of the bases. In such an embodiment, the method of detecting methylation, and in which case the region (iv) is set as a highly methylated region and in which case the region (iv) is set as a low methylated region are the same as those described above for the region (i).

In addition, in one embodiment of the present invention, for example, the frequency of methylation of the region (v) can be measured by detecting methylation of base(s) in at least one site (preferably 2 or more sites, more preferably 3 or more sites, more preferably 4 or more sites, and further preferably 5 sites) selected from the group consisting of the following (v-1) to (v-5):

(v-1) a base at position 186048714 of chromosome 4
(v-2) a base at position 186048907 of chromosome 4
(v-3) a base at position 186049687 of chromosome 4
(v-4) a base at position 186049926 of chromosome 4
(v-5) a base at position 186050047 of chromosome 4.

In particular, it is preferable to detect methylation of at least (v-3) among the positions of the bases. In such an embodiment, the method of detecting methylation, and in which case the region (v) is set as a highly methylated region and in which case the region (v) is set as a low methylated region are the same as those described above for the region (i).

In addition, in one embodiment of the present invention, for example, when the frequency of methylation of the region (vi) is measured, the frequency of methylation of the region (vi) can be measured by detecting methylation of base(s) in at least one site (preferably 2 or more sites, more preferably 7 or more sites, more preferably 10 or more sites, and further preferably 13 sites) selected from the group consisting of the following (vi-1) to (vi-13):

(vi-1) a base at position 17216679 of chromosome 5
(vi-2) a base at position 17216922 of chromosome 5
(vi-3) a base at position 17217093 of chromosome 5
(vi-4) a base at position 17217877 of chromosome 5
(vi-5) a base at position 17218089 of chromosome 5
(vi-6) a base at position 17218278 of chromosome 5
(vi-7) a base at position 17218308 of chromosome 5
(vi-8) a base at position 17218547 of chromosome 5
(vi-9) a base at position 17218778 of chromosome 5
(vi-10) a base at position 17219020 of chromosome 5
(vi-11) a base at position 17219128 of chromosome 5
(vi-12) a base at position 17219226 of chromosome 5
(vi-13) a base at position 17219239 of chromosome 5.

In particular, it is preferable to detect methylation of at least (vi-2) among the positions of the bases. In such an embodiment, the method of detecting methylation, and in which case the region (vi) is set as a highly methylated region and in which case the region (vi) is set as a low methylated region are the same as those described above for the region (i).

In addition, in one embodiment of the present invention, for example, when the frequency of methylation of the region (vii) is measured, the frequency of methylation of the region (vii) can be measured by detecting methylation of base(s) in at least one site (preferably 2 or more sites, further preferably 3 sites) selected from the group consisting of (vii-1) to (vii-3): (vii-1) a base at position 37663982 of chromosome 6

(vii-2) a base at position 37664451 of chromosome 6
(vii-3) a base at position 37664538 of chromosome 6.

In particular, it is preferable to detect methylation of at least (vii-2) among the positions of the bases. In such an embodiment, the method of detecting methylation, and in which case the region (vii) is set as a highly methylated region and in which case the region (vii) is set as a low methylated region are the same as those described above for the region (i).

In addition, in one embodiment of the present invention, for example, when the frequency of methylation of the region (viii) is measured, the frequency of methylation of the region (viii) can be measured by detecting methylation of base(s) in at least one site (preferably 2 or more sites, more preferably 5 or more sites, more preferably 7 or more sites, and further preferably 9 sites) selected from the group consisting of the following (viii-1) to (viii-9):

(viii-1) a base at position 39281183 of chromosome 6
(viii-2) a base at position 39281421 of chromosome 6
(viii-3) a base at position 39281450 of chromosome 6

(viii-4) a base at position 39281541 of chromosome 6
(viii-5) a base at position 39281694 of chromosome 6
(viii-6) a base at position 39281885 of chromosome 6
(viii-7) a base at position 39282164 of chromosome 6
(viii-8) a base at position 39282316 of chromosome 6
(viii-9) a base at position 39282331 of chromosome 6.

In particular, it is preferable to detect methylation of at least (viii-5) among the positions of the bases. In such an embodiment, the method of detecting methylation, and in which case the region (viii) is set as a highly methylated region and in which case the region (viii) is set as a low methylated region are the same as those described above for the region (i).

In addition, in one embodiment of the present invention, for example, when the frequency of methylation of the region (ix) is measured, the frequency of methylation of the region (ix) can be measured by detecting methylation of base(s) in at least one site (preferably 2 or more sites, more preferably 12 or more sites, more preferably 18 or more sites, and further preferably 23 sites) selected from the group consisting of the following (ix-1) to (ix-23):

(ix-1) a base at position 42273390 of chromosome 7
(ix-2) a base at position 42275601 of chromosome 7
(ix-3) a base at position 42275813 of chromosome 7
(ix-4) a base at position 42275870 of chromosome 7
(ix-5) a base at position 42275872 of chromosome 7
(ix-6) a base at position 42276004 of chromosome 7
(ix-7) a base at position 42276814 of chromosome 7
(ix-8) a base at position 42276816 of chromosome 7
(ix-9) a base at position 42276819 of chromosome 7
(ix-10) a base at position 42276848 of chromosome 7
(ix-11) a base at position 42276881 of chromosome 7
(ix-12) a base at position 42276890 of chromosome 7
(ix-13) a base at position 42276941 of chromosome 7
(ix-14) a base at position 42276981 of chromosome 7
(ix-15) a base at position 42277044 of chromosome 7
(ix-16) a base at position 42277066 of chromosome 7
(ix-17) a base at position 42277071 of chromosome 7
(ix-18) a base at position 42277347 of chromosome 7
(ix-19) a base at position 42277375 of chromosome 7
(ix-20) a base at position 42277394 of chromosome 7
(ix-21) a base at position 42277410 of chromosome 7
(ix-22) a base at position 42277807 of chromosome 7
(ix-23) a base at position 42277950 of chromosome 7.

In particular, it is preferable to detect methylation of at least (ix-8) among the positions of the bases. In such an embodiment, the method of detecting methylation, and in which case the region (ix) is set as a highly methylated region and in which case the region (ix) is set as a low methylated region are the same as those described above for the region (i).

In addition, in one embodiment of the present invention, for example, when the frequency of methylation of the region (x) is measured, the frequency of methylation of the region (x) can be measured by detecting methylation of base(s) in at least one site (preferably 2 or more sites, more preferably 3 or more sites, and further preferably 4 sites) selected from the group consisting of the following (x-1) to (x-4):

(x-1) a base at position 13039715 of chromosome 10
(x-2) a base at position 13041989 of chromosome 10
(x-3) a base at position 13043313 of chromosome 10
(x-4) a base at position 13043421 of chromosome 10.

In particular, it is preferable to detect methylation of at least (x-3) among the positions of the bases. In such an embodiment, the method of detecting methylation, and in which case the region (x) is set as a highly methylated region and in which case the region (x) is set as a low methylated region are the same as those described above for the region (i).

In addition, in one embodiment of the present invention, for example, when the frequency of methylation of the region (xi) is measured, the frequency of methylation of the region (xi) can be measured by detecting methylation of base(s) in at least one site (preferably 2 or more sites, more preferably 4 or more sites, more preferably 5 or more sites, and further preferably 6 sites) selected from the group consisting of the following (xi-1) to (xi-6):

(xi-1) a base at position 81664401 of chromosome 10
(xi-2) a base at position 81664567 of chromosome 10
(xi-3) a base at position 81664583 of chromosome 10
(xi-4) a base at position 81664698 of chromosome 10
(xi-5) a base at position 81664955 of chromosome 10
(xi-6) a base at position 81665075 of chromosome 10.

In particular, it is preferable to detect methylation of at least (xi-3) among the positions of the bases. In such an embodiment, the method of detecting methylation, and in which case the region (xi) is set as a highly methylated region and in which case the region (xi) is set as a low methylated region are the same as those described above for the region (i).
In addition, in one embodiment of the present invention, for example, when the frequency of methylation of the region (xii) is measured, the frequency of methylation of the region (xii) can be measured by detecting methylation of base(s) in at least one site (preferably 2 or more sites, more preferably 3 or more sites, more preferably 4 or more sites, and further preferably 6 sites) selected from the group consisting of the following (xii-1) to (xii-6):

(xii-1) a base at position 88126287 of chromosome 10
(xii-2) a base at position 88126291 of chromosome 10
(xii-3) a base at position 88126299 of chromosome 10
(xii-4) a base at position 88126306 of chromosome 10
(xii-5) a base at position 88126853 of chromosome 10
(xii-6) a base at position 88127188 of chromosome 10.

In particular, it is preferable to detect methylation of at least (xii-4) among the positions of the bases. In such an embodiment, the method of detecting methylation, and in which case the region (xii) is set as a highly methylated region and in which case the region (xii) is set as a low methylated region are the same as those described above for the region (i).
In addition, in one embodiment of the present invention, for example, when the frequency of methylation of the region (xiii) is measured, the frequency of methylation of the region (xiii) can be measured by detecting methylation of base(s) in at least one site (preferably 2 or more sites, more preferably 5 or more sites, and more preferably 7 sites) selected from the group consisting of the following (xiii-1) to (xiii-7):

(xiii-1) a base at position 61595807 of chromosome 11
(xiii-2) a base at position 61595956 of chromosome 11
(xiii-3) a base at position 61596068 of chromosome 11
(xiii-4) a base at position 61596307 of chromosome 11
(xiii-5) a base at position 61596333 of chromosome 11
(xiii-6) a base at position 61596405 of chromosome 11
(xiii-7) a base at position 61596626 of chromosome 11.

In particular, it is preferable to detect methylation of at least (xiii-1) among the positions of the bases. In such an embodiment, the method of detecting methylation, and in which case the region (xiii) is set as a highly methylated region and in which case the region (xiii) is set as a low methylated region are the same as those described above for the region (i).
In addition, in one embodiment of the present invention, for example, when the frequency of methylation of the region (xiv) is measured, the frequency of methylation of the region (xiv) can be measured by detecting methylation of base(s) in at least one site (preferably 2 or more sites, more preferably 5 or more sites, more preferably 8 or more sites, and further preferably 10 sites) selected from the group consisting of the following (xiv-1) to (xiv-10):

(xiv-1) a base at position 23820337 of chromosome 14
(xiv-2) a base at position 23821149 of chromosome 14
(xiv-3) a base at position 23821229 of chromosome 14
(xiv-4) a base at position 23821435 of chromosome 14
(xiv-5) a base at position 23821445 of chromosome 14

(xiv-6) a base at position 23821570 of chromosome 14
(xiv-7) a base at position 23821596 of chromosome 14
(xiv-8) a base at position 23821902 of chromosome 14
(xiv-9) a base at position 23822017 of chromosome 14
(xiv-10) a base at position 23822265 of chromosome 14.

In particular, it is preferable to detect methylation of at least (xiv-9) among the positions of the bases. In such an embodiment, the method of detecting methylation, and in which case the region (xiv) is set as a highly methylated region and in which case the region (xiv) is set as a low methylated region are the same as those described above for the region (i).

In addition, in one embodiment of the present invention, for example, when the frequency of methylation of the region (xv) is measured, the frequency of methylation of the region (xv) can be measured by detecting methylation of base(s) in at least one site (preferably 2 or more sites, more preferably 6 or more sites, more preferably 8 or more sites, and further preferably 11) selected from the group consisting of the following (xv-1) to (xv-11):

(xv-1) a base at position 44895317 of chromosome 17
(xv-2) a base at position 44896017 of chromosome 17
(xv-3) a base at position 44896080 of chromosome 17
(xv-4) a base at position 44896147 of chromosome 17
(xv-5) a base at position 44896162 of chromosome 17
(xv-6) a base at position 44896166 of chromosome 17
(xv-7) a base at position 44896168 of chromosome 17
(xv-8) a base at position 44896212 of chromosome 17
(xv-9) a base at position 44896223 of chromosome 17
(xv-10) a base at position 44896424 of chromosome 17
(xv-11) a base at position 44896748 of chromosome 17.

In particular, it is preferable to detect methylation of at least (xv-10) among the positions of the bases. In such an embodiment, the method of detecting methylation, and in which case the region (xv) is set as a highly methylated region and in which case the region (xv) is set as a low methylated region are the same as those described above for the region (i).

In addition, in one embodiment of the present invention, for example, when the frequency of methylation of the region (xvi) is measured, the frequency of methylation of the region (xvi) can be measured by detecting methylation of base(s) in at least one site (preferably 2 or more sites, more preferably 4 or more sites, more preferably 6 or more sites, and further preferably 8 sites) selected from the group consisting of the following (xvi-1) to (xvi-8):

(xvi-1) a base at position 39520228 of chromosome 19
(xvi-2) a base at position 39521931 of chromosome 19
(xvi-3) a base at position 39522418 of chromosome 19
(xvi-4) a base at position 39522548 of chromosome 19
(xvi-5) a base at position 39522747 of chromosome 19
(xvi-6) a base at position 39522944 of chromosome 19
(xvi-7) a base at position 39523083 of chromosome 19
(xvi-8) a base at position 39523158 of chromosome 19.

In particular, it is preferable to detect methylation of at least (xvi-4) among the positions of the bases. In such an embodiment, the method of detecting methylation, and in which case the region (xvi) is set as a highly methylated region and in which case the region (xvi) is set as a low methylated region are the same as those described above for the region (i).

By detecting methylation of a plurality of CpG sites among the CpG sites comprised in each region by the above method, sensitivity of chemotherapy against colorectal cancer can be accurately tested even when the methylation frequency of only one region among the regions (i) to (xvi) is measured. In that case, when the measured region is a highly methylated region based on the above-described criteria, the patient can be determined as a patient with highly methylated, having no sensitivity of chemotherapy against colorectal cancer. In addition, when the measured region is a low methylated region, the patient can be determined as a patient with low methylated, having sensitivity of chemotherapy against colorectal cancer. Further, in such an embodiment, even when the frequency is measured by detecting methylation of base(s) at a relatively small number of positions (for example, base(s) at 1 to 3 sites, 1 to 2 sites, or 1 site per region) among the CpG sites comprised in each region, when the frequency of methylation of two or more regions among the regions (i) to (xvi) is measured and regions of more than or equal to a predetermined ratio (for example, half or more, 60% or more, or 70% or more) of the regions are highly methylated regions, sensitivity can be accurately determined by determining the patient as a patient with highly methylated, having no sensitivity of chemotherapy against colorectal cancer. Furthermore, in such an embodiment, when methylation of two or more regions among the regions (i) to (xvi) is detected and regions of less than a predetermined ratio (for example, less than half, less than 40%, or less than 30%) of the regions are highly methylated regions, sensitivity can be accurately determined by determining the patient as a patient with low methylated, having sensitivity of chemotherapy against colorectal cancer.

In addition, in another preferred embodiment of the present invention, methylation of the following CpG sites is preferably used as an indicator:

In this embodiment, it is possible to detect methylation in at least one site (preferably 3 or more sites, more preferably 5 or more sites, further preferably 7 or more sites, most preferably 10 sites) selected from the group consisting of bases at positions 207308141, 207308149, 207308153, 207308172, 207308177, 207308181, 207308185, 207308244, 207308247, and 207308267 of chromosome 2. In this embodiment, it is preferable to detect methylation at at least position 207308244 of chromosome 2. When bases of more than or equal to predetermined ratio $Ta_1$ or more among the above bases are methylated, the region can be determined as a highly methylated region. The reference value $Ta_1$ can be appropriately set in the range of, for example, $0.80 \leq Ta_1$, more preferably 0.90 ≤ Tai, and further preferably 0.95 ≤ Tai.

In this embodiment, it is possible to detect methylation in at least one site (preferably 3 or more sites, more preferably 6 or more sites, further preferably 9 or more sites, most preferably 12 sites) selected from the group consisting of bases at positions 241758885, 241758889, 241758896, 241758901, 241758919, 241758922, 241758931, 241758936, 241758940, 241759003, 241759005, and 241759009 of chromosome 2. In this embodiment, it is preferable to detect methylation at at least position 241758901 of chromosome 2. When bases of more than or equal to predetermined ratio Tai or more among the bases to be detected are methylated, the region can be determined as a highly methylated region. The reference value $Ta_1$ can be set in the same manner as in the above-described embodiment (the same applies hereinafter).

In this embodiment, it is possible to detect methylation in at least one site (preferably 3 or more sites, more preferably 5 or more sites, further preferably 7 or more sites, most preferably 9 sites) selected from the group consisting of bases at positions 150804417, 150804420, 150804474, 150804479, 150804486, 150804490, 150804496, 150804504, and 150804507 of chromosome 3. In this embodiment, it is preferable to detect methylation at at least position 150804490 of chromosome 3. When bases of more than or equal to predetermined ratio Tai or more among the bases to be detected are methylated, the region can be determined as a highly methylated region.

In this embodiment, it is possible to detect methylation in at least one site (preferably 2 or more sites, more preferably 4 or more sites, further preferably 6 or more sites, most preferably 8 sites) selected from the group consisting of bases at positions 141348224, 141348232, 141348272, 141348282, 141348289, 141348307, 141348318, and 141348324 of chromosome 4. In this embodiment, it is preferable to detect methylation at at least position 141348307 of chromosome 4. When bases of more than or equal to predetermined ratio Tai or more among the bases to be detected are methylated, the region can be determined as a highly methylated region.

In this embodiment, it is possible to detect methylation in at least one site (preferably 2 or more sites, more preferably 4 or more sites, further preferably 6 or more sites, most preferably 8 sites) selected from the group consisting of bases at positions 186049623, 186049636, 186049659, 186049670, 186049685, 186049687, 186049689, and 186049705 of chromosome 4. In this embodiment, it is preferable to detect methylation at at least position 186049687 of chromosome 4. When bases of more than or equal to predetermined ratio Tai or more among the bases to be detected are methylated, the region can be determined as a highly methylated region.

In this embodiment, it is possible to detect methylation in at least one site (preferably 3 or more sites, more preferably 6 or more sites, further preferably 9 or more sites, most preferably 10 sites) selected from the group consisting of bases at positions 17216904, 17216916, 17216922, 17216940, 17216949, 17216952, 17217003, 17217011, 17217017, and 17217023 of chromosome 5. In this embodiment, it is preferable to detect methylation at at least position 17216922 of chromosome 5. When bases of more than or equal to predetermined ratio $Ta_1$ or more among the bases to be detected are methylated, the region can be determined as a highly methylated region.

In this embodiment, it is possible to detect methylation in at least one site (preferably 2 or more sites, more preferably 4 or more sites, further preferably 5 or more sites, most preferably 6 sites) selected from the group consisting of bases at positions 37664352, 37664366, 37664419, 37664424, 37664451, and 37664460 of chromosome 6. In this embodiment, it is preferable to detect methylation at at least position 37664451 of chromosome 6. When bases of more than or equal to predetermined ratio $Ta_1$ or more among the bases to be detected are methylated, the region can be determined as a highly methylated region.

In this embodiment, it is possible to detect methylation in at least one site (preferably 3 or more sites, more preferably 6 or more sites, further preferably 9 or more sites, most preferably 10 sites) selected from the group consisting of bases at positions 39281692, 39281694, 39281723, 39281729, 39281735, 39281738, 39281797, 39281806, 39281808, and 39281813 of chromosome 6. In this embodiment, it is preferable to detect methylation at at least position 39281694 of chromosome 6. When bases of more than or equal to predetermined ratio $Ta_1$ or more among the bases to be detected are methylated, the region can be determined as a highly methylated region.

In this embodiment, it is possible to detect methylation in at least one site (preferably 3 or more sites, more preferably 6 or more sites, further preferably 9 or more sites, most preferably 12 sites) selected from the group consisting of bases at positions 42276764, 42276767, 42276769, 42276783, 42276810, 42276814, 42276816, 42276819, 42276825, 42276852, 42276862, and 42276874 of chromosome 7. In this embodiment, it is preferable to detect methylation at at least position 42276816 of chromosome 7. When bases of more than or equal to predetermined ratio Tai or more among the bases to be detected are methylated, the region can be determined as a highly methylated region.

In this embodiment, it is possible to detect methylation in at least one site (preferably 2 or more sites, more preferably 4 or more sites, further preferably 6 or more sites, most preferably 7 sites) selected from the group consisting of bases at positions 13043231, 13043247, 13043285, 13043288, 13043313, 13043321, and 13043333 of chromosome 10. In this embodiment, it is preferable to detect methylation at at least position 13043313 of chromosome 10. When bases of more than or equal to predetermined ratio $Ta_1$ or more among the bases to be detected are methylated, the region can be determined as a highly methylated region.

In this embodiment, it is possible to detect methylation in at least one site (preferably 3 or more sites, more preferably 5 or more sites, further preferably 7 or more sites, most preferably 9 sites) selected from the group consisting of bases at positions 81664567, 81664573, 81664583, 81664598, 81664600, 81664606, 81664614, 81664636, and 81664656 of chromosome 10. In this embodiment, it is preferable to detect methylation at at least position 81664583 of chromosome 10. When bases of more than or equal to predetermined ratio Tai or more among the bases to be detected are methylated, the region can be determined as a highly methylated region.

In this embodiment, it is possible to detect methylation in at least one site (preferably 3 or more sites, more preferably 5 or more sites, further preferably 7 or more sites, most preferably 9 sites) selected from the group consisting of bases at positions 88126243, 88126250, 88126259, 88126285, 88126287, 88126291, 88126299, 88126306, and 88126310 of chromosome 10. In this embodiment, it is preferable to detect methylation at at least position 88126306 of chromosome 10. When bases of more than or equal to predetermined ratio Tai or more among the bases to be detected are methylated, the region can be determined as a highly methylated region.

In this embodiment, it is possible to detect methylation in at least one site (preferably 2 or more sites, more preferably 4 or more sites, further preferably 6 or more sites, most preferably 8 sites) selected from the group consisting of bases at positions 61595796, 61595807, 61595815, 61595818, 61595820, 61595829, 61595843, and 61595852 of chromosome 11. In this embodiment, it is preferable to detect methylation at at least position 61595807 of chromosome 11. When bases of more than or equal to predetermined ratio $Ta_1$ or more among the bases to be detected are methylated, the region can be determined as a highly methylated region.

In this embodiment, it is possible to detect methylation in at least one site (preferably 2 or more sites, more preferably 4 or more sites, further preferably 6 or more sites, most preferably 7 sites) selected from the group consisting of bases at positions 23822015, 23822017, 23822043, 23822054, 23822073, 23822075, and 23822079 of chromosome 14. In this embodiment, it is preferable to detect methylation at at least position 23822017 of chromosome 14. When bases of more than or equal to predetermined ratio $Ta_1$ or more among the bases to be detected are methylated, the region can be determined as a highly methylated region.

In this embodiment, it is possible to detect methylation in at least one site (preferably 2 or more sites, more preferably 4 or more sites, further preferably 6 or more sites, most preferably 8 sites) selected from the group consisting of bases at positions 44896314, 44896316, 44896324, 44896386, 44896390, 44896401, 44896424, 44896443, and 44896450 of chromosome 17. In this embodiment, it is preferable to detect methylation at at least position 44896424 of chromosome 17. When bases of more than or equal to predetermined ratio Tai or more among the bases to be detected are methylated, the region can be determined as a highly methylated region.

In this embodiment, it is possible to detect methylation in at least one site (preferably 3 or more sites, more preferably 5 or more sites, further preferably 8 or more sites, most preferably 11 sites) selected from the group consisting of bases at positions 39522493, 39522510, 39522533, 39522537, 39522548, 39522550, 39522552, 39522582, 39522587, 39522591, and 39522608 of chromosome 19. In this embodiment, it is preferable to detect methylation at at least position 39522548 of chromosome 19. When bases of more than or equal to predetermined ratio Tai or more among the bases to be detected are methylated, the region can be determined as a highly methylated region.

Also in the present embodiment, the frequency of methylation in the region to be measured can be determined by a method of calculating a ΔCt value using real-time PCR, or the like. Specifically, for example, a primer and a probe set are designed so that the region to be measured can be amplified, real-time PCR is performed to calculate a ΔCt value, and when ΔCt is less than a predetermined value Tb, the region can be set as a highly methylated region. In addition, the ΔCt value is calculated by the above method, and when ΔCt is equal to or more than the predetermined value Tb, the region can be set as a low methylated region. The reference value Tb can be set in the same manner as in the above-described embodiment.

In addition, in another preferred embodiment of the present invention, methylation of the following CpG sites is preferably used as an indicator:
In this embodiment, it is possible to use, as an indicator, methylation in at least one site selected from the group consisting of CpG sites comprised in:

a region at positions 207308134 to 207308268 of chromosome 2
a region at positions 241758885 to 241759011 of chromosome 2
a region at positions 150804402 to 150804508 of chromosome 3
a region at positions 141348215 to 141348329 of chromosome 4
a region at positions 186049616 to 186049706 of chromosome 4
a region at positions 17216901 to 17217025 of chromosome 5
a region at positions 37664344 to 37664472 of chromosome 6
a region at positions 39281671 to 39281814 of chromosome 6
a region at positions 42276764 to 42276875 of chromosome 7
a region at positions 13043227 to 13043334 of chromosome 10
a region at positions 81664566 to 81664662 of chromosome 10
a region at positions 88126243 to 88126334 of chromosome 10
a region at positions 61595787 to 61595864 of chromosome 11
a region at positions 23821996 to 23822092 of chromosome 14
a region at positions 44896309 to 44896451 of chromosome 17
a region at positions 39522493 to 39522609 of chromosome 19

In this embodiment, length of the region to be measured is not particularly limited, but can be appropriately set in the range of, for example, 75 to 200 bases, preferably 90 to 160 bases, and more preferably 100 to 150 bases.
Also in the present embodiment, the frequency of methylation in the region to be measured can be determined by a method of calculating a ΔCt value using real-time PCR, or the like. Specifically, for example, a primer and a probe set are designed so that the region to be measured can be amplified, real-time PCR is performed to calculate a ΔCt value, and when ΔCt is less than a predetermined value Tb, the region can be set as a highly methylated region. In addition, the ΔCt value is calculated by the above method, and when ΔCt is equal to or more than the predetermined value Tb, the region can be set as a low methylated region. The reference value Tb can be set in the same manner as in the above-described embodiment. The frequency of methylation can also be determined based on the method of calculating a β value using a bead array.
In a preferred embodiment of the present invention, examples of probes and primer sets for measuring the frequency of methylation of each region include the following:

Region (i)
F-Primer TAGTATTCGAAGTTTCGTTCG (SEQ ID NO: 1)
R-Primer GAACCTAAAAATACTAAAACGACG (SEQ ID NO: 2)
Probe AAACGAACGTACGCCCCGC (SEQ ID NO: 3)
Region (ii)
F-Primer CGGTCGTGTAACGAAGC (SEQ ID NO: 4)
R-Primer CCGAACGCGAACAACTA (SEQ ID NO: 5)
Probe CAAACGAACGAAACGAAAACTACGACG (SEQ ID NO: 6)
Region (iii)
F-Primer AGTAGAGGAGGTAGGCGTC (SEQ ID NO: 7)
R-Primer CGACGAATACTCGACTACG (SEQ ID NO: 8)
Probe CTCGAACCCCGAAACGCAC (SEQ ID NO: 9)
Region (iv)
F-Primer GAGTCGAGGTCGTTATTTTAGC (SEQ ID NO: 10)
R-Primer AATAAACAACGCCCAAACG (SEQ ID NO: 11)
probe CCCCGAACTCAAACGTAAAACG (SEQ ID NO: 12)
Region (v)
F-Primer GGGGAAAGTTTTTATCGC (SEQ ID NO: 13)
R-Primer CTAATAACGTTACCCACAACCG (SEQ ID NO: 14)
probe CCCGAAAAAACTACGAAATAAAACTAACCG (SEQ ID NO: 15)
Region (vi)
F-Primer TCGATTACGATTTCGTTATTTC (SEQ ID NO: 16)
R-Primer ATACGAATACAAATACGAAACCG (SEQ ID NO: 17)

probe CAACTCGTTTACAACGACGCAACC (SEQ ID NO: 18)
Region (vii)
F-Primer TTTGTAAGAGGCGTAAAGTGC (SEQ ID NO: 19)
R-Primer CTATATTCCGAAACAACTAACTCG (SEQ ID NO: 20) probe CATCCACTCCAAAATCCGAATCG (SEQ ID NO: 21)
Region (viii)
F-Primer GTTTTTTAATTTTTTGGTTTTCGC (SEQ ID NO: 22)
R-Primer CGAACCGCGAAATATACGA (SEQ ID NO: 23)
probe AACGTCGAAAACGCAAACGC (SEQ ID NO: 24)
Region (ix)
F-Primer GAAATTGATGTCGGAGTTGTAC (SEQ ID NO: 25)
R-Primer GACGCGAATAAATAAAAACG (SEQ ID NO: 26)
Probe TACGAACGCGCCGATAACGC (SEQ ID NO: 27)
Region (x)
F-Primer ATTACGGTGTGGGAGTGGTAC (SEQ ID NO: 28)
R-Primer CGAAATACTATACGACCAAACG (SEQ ID NO: 29)
probe CAACATACTAAAAATACCCGCCGACTC (SEQ ID NO: 30)
Region (xi)
F-Primer AGATTCGTTAATAGTTGAGTGTAGGC (SEQ ID NO: 31)
R-Primer CCGAAAACGAAATAAAACG (SEQ ID NO: 32)
probe CACGCGAACCCGATAAAACG (SEQ ID NO: 33)
Region (xii)
F-Primer GTTTTTCGGGAGAGTCGAG (SEQ ID NO: 34)
R-Primer AACTAAACTAAACTAAACTAAAACGAACG (SEQ ID NO: 35)
probe CAAAACGCAAAAACGAACGCG (SEQ ID NO: 36)
Region (xiii)
F-Primer ATGTTGTTTGTCGATTGTGAC (SEQ ID NO: 37)
R-Primer AAAAACAACCGTCTATACAACG (SEQ ID NO: 38)
probe AACCGACGCGAAAAAACCG (SEQ ID NO: 39)
Region (xiv)
F-Primer AGATTAAGGAGGCGTTCGC (SEQ ID NO: 40)
R-Primer CTATCCTCTAACTCAATTACGCG (SEQ ID NO: 41)
Probe CAACTCGAAAAAATCCCGCTCTACAAC (SEQ ID NO: 42)
Region (xv)
F-Primer GGTTTTCGTTAGTAAAGGAGTTATTC (SEQ ID NO: 43)
R-Primer AAAAACGCGATAAACCGAAC (SEQ ID NO: 44)
probe CTTTAACCCGAACGTTCACATAACGAA (SEQ ID NO: 45)
Region (xvi)
F-Primer GTAGTTGTTAGTTTTTCGTTCGTAAC (SEQ ID NO: 46)
R-Primer GAAATCCCACCTTAACCGC (SEQ ID NO: 47)
probe TTACGTCCGATAAATCTACGCGCG (SEQ ID NO: 48)

In the present specification, a primer set of the forward primer and the reverse primer for the region (i) is referred to as primer set 1. Similarly, primer sets of the forward primer and the reverse primer for the regions (ii) to (xvi) are each referred to as primer sets 2 to 16. The probe for the region (i) is referred to as probe 1. Similarly, the probes for the regions (ii) to (xvi) are each referred to as probes 2 to 16.

Therefore, in a preferred embodiment of the present invention, the region to be measured can be set so as to be detected using at least one of the probes 1 to 16 and/or at least one of the primer sets 1 to 16. Alternatively, in a preferred embodiment of the present invention, the region to be measured can be set so as to be detected using at least one of the probes 1 to 16 and/or at least one of the primer sets 1 to 16 (or at least one of primers constituting each primer of the primer sets 1 to 16). Alternatively, in a preferred embodiment of the present invention, it is preferable that at least a part of the CpG site as an indicator is comprised in a region where any of the primers constituting each primer of the primer sets 1 to 16 is hybridized after bisulfite treatment. Alternatively, in a preferred embodiment of the present invention, it is preferable that the CpG site to be used as an indicator is located in a region to which any one of the primer sets 1 to 16 and the probes 1 to 16 hybridizes in DNA obtained by bisulfite treatment of DNA containing the CpG site that is methylated. In the present invention, the term "hybridize" means to hybridize under stringent conditions usually set in the technical field of the present invention. For example, it means to hybridize under the conditions of Example 2 of the present application. Stringency upon hybridization is known to be functions of temperature, salt concentration, primer chain length, GC content of primer nucleotide sequence, and concentration of chaotropic agent in a hybridization buffer, and those skilled in the art can appropriately adjust stringency in consideration of the above conditions with reference to known information such as Sambrook, J. et al. (1998) Molecular Cloning: A Laboratory Manual (2nd ed.), ColdSpringHarborLaboratory Press, New York. In the present invention, the region to which an oligonucleotide hybridizes in DNA obtained by bisulfite treatment of DNA containing a CpG site that is methylated refers to a region to which an oligonucleotide hybridizes in a region corresponding to the region in a resultant product of bisulfite treatment of DNA containing the region. By the bisulfite treatment, unmethylated cytosine contained in the region is converted into uracil, and methylated cytosine is not converted. The above-described primer sets 1 to 16 and probes 1 to 16 are designed such that all cytosines in the CpG site comprised in the region to be hybridized are methylated (thus not converted by bisulfite treatment), cytosines not present in the CpG site are not methylated (thus converted into uracils by bisulfite treatment), and these are hybridized after bisulfite treatment of the region. As described above, in a typical embodiment, based on the base sequence of a certain region in the reference sequence, when cytosine in the CpG site in the reference base sequence is left as it is and a certain oligonucleotide hybridizes to a base sequence obtained by converting all cytosines not in the CpG site to uracils, the region can be set as a region "to which an oligonucleotide hybridizes in DNA obtained by bisulfite treatment of DNA containing a CpG site that is methylated".

In the present invention, a "method for testing for sensitivity of chemotherapy against colorectal cancer, using methylation of regions (a) to (c) as an indicator" may include not only a method using methylation of only the regions (a) to (c) as an indicator, but also a method comprising a step of detecting methylation of the regions (a) to (c) and other regions in a range in which test results do not differ. For example, a "method for testing for sensitivity of chemotherapy against colorectal cancer, using methylation of the regions (i) to (v) as an indicator" may include not only a method using methylation of only the regions (i) to (v) as an indicator, but also a method comprising a step of detecting methylation of the regions (i) to (v) and other regions ("other regions" may be regions other than the regions (vi) to (xvi)) in a range in which test results do not differ. Therefore, the method of the present invention can also include a method comprising a step of detecting methylation in at least one site selected from the group consisting of CpG sites comprised in the regions (i) to (xvi) and detecting methylation of a region that does not substantially affect the test result.

(2. Method for treating colorectal cancer patient)

The present invention provides a method for treating a colorectal cancer patient, the method comprising the steps of determining sensitivity of chemotherapy against colorectal cancer using methylation in at least one site selected from the group consisting of CpG sites comprised in the regions (i) to (xvi) in DNA collected from a colorectal cancer patient, as an indicator, and administering the chemotherapy to a colorectal cancer patient determined to have sensitivity in the step.

The chemotherapy to be administered to a colorectal cancer patient determined to have sensitivity of chemotherapy against colorectal cancer in the above sensitivity determination is not particularly limited, but for example, the chemotherapy can be performed by administering an effective amount of the drug used for the chemotherapy against colorectal cancer to a colorectal cancer patient.

Administration route of the drug is not particularly limited, and may be oral administration or parenteral administration. Examples of the parenteral administration include oral administration, airway administration, rectal administration, subcutaneous administration, intramuscular administration, intravenous administration, and the like.

Preparation form of the drug is not particularly limited, and examples thereof include various preparation forms such as oral administration agents such as tablets, pills, capsules, powders, granules, and syrups; and parenteral administration agents such as injections (intravenous injection, intramuscular injection, local injection, and the like), gargles, drops, external preparations (ointments, creams, patches, inhalants), and suppositories.

In the present invention, the dose of the drug to be used for chemotherapy varies depending on the route of administration, age, body weight, symptoms, and the like of the patient, and cannot be unconditionally specified. However, the dose of the active ingredient may be an amount such that the daily dose for an adult is usually about 5000 mg or less, preferably about 1000 mg or less, and more preferably 500 mg or less. The lower limit of the dose is also not particularly limited, and for example, as the dose of the active ingredient, the daily dose for an adult can be appropriately set in the range of usually 1 mg or more, preferably 10 mg or more, and more preferably 100 mg or more. In the case of administration once a day, the above amount may be contained in one preparation, and in the case of administration three times a day, 1/3 of the above amount may be contained in one preparation.

(3. Other embodiments)

Although the present application has been described using specific embodiments, the present invention is not limited to such embodiments. As described above, the present invention is based on the novel finding that the regions (i) to (xvi) are useful as an indicator of sensitivity of chemotherapy against colorectal cancer. Therefore, the present invention also includes, for example, the following embodiments: a method for assisting determination of sensitivity of chemotherapy against colorectal cancer, the method using, as an indicator, methylation in at least one site selected from the group consisting of CpG sites comprised in the following regions (i) to (xvi) in DNA collected from a colorectal cancer patient; a method in which methylation in at least one site selected from the group consisting of CpG sites comprised in the regions (i) to (xvi) in DNA collected from a colorectal cancer patient is used as an indicator for determining sensitivity of chemotherapy against colorectal cancer; a kit for determining sensitivity of chemotherapy against colorectal cancer, including a component for detecting methylation in at least one site selected from the group consisting of CpG sites comprised in the regions (i) to (xvi) in DNA collected from a colorectal cancer patient; and a program for causing a computer to implement a first function of acquiring a score indicating a frequency of methylation in at least one site selected from the group consisting of the regions (i) to (xvi) in DNA collected from a colorectal cancer patient, a second function of determining a region as a highly methylated region when a score indicating the frequency of methylation acquired by the first function is equal to or less than a value preset for each region, and a third function of determining that there is no sensitivity of chemotherapy against colorectal cancer when half or more of the regions (i) to (xvi) for which the score is acquired by the first function are determined as highly methylated regions by the second function.

Descriptions of DNA collected from a colorectal cancer patient, chemotherapy, each region, and the like in these embodiments; details of the method of detecting methylation of each region, and the like are as described above. In the kit embodiment, examples of component for detecting methylation include a probe, a primer set, and the like used in the method described above. Also, the kit of the present invention can contain other components as necessary. Examples of the other components include, but are not limited to, tools for collecting a sample (for example, syringe), and the like. The kit can also contain a written description of a procedure for performing the test method, and the like.

In addition, specific embodiments of the present invention will be described more specifically below with reference to Examples, but it is also apparent that the present invention is not limited to such Examples.

## Examples

Reference Example 1: Comprehensive DNA methylation analysis using 97 cases of colorectal cancer

Comprehensive DNA methylation analysis by Infinium 450K (Illumina) was performed using formalin-fixed paraffin-embedded tissues (FFPE specimens) of colorectal cancer tumor tissues surgically removed from 97 cases of colorectal cancer having a history of using an anti-EGFR antibody drug. Subject cases were cases in which no mutation was observed in KRAS exon 2 by Sanger method.

β values (methylated probes/methylated probes + unmethylated probes) were calculated for each of 486,428 targeted CpG sites, and unsupervised hierarchical cluster analysis was performed using 3,163 probes with a standard deviation of a distribution of β values exceeding 0.25 according to the method described in Patent Literature 1.

As a result, the analysis target disease cases were classified into 2 groups of a highly-methylated colorectal cancer (HMCC) group (34 cases) with a high methylation level and a low-methylated colorectal cancer (LMCC) group (63 cases) with a low methylation level.

## Example 1

Statistical studies of the β values of each CpG site in 63 cases in the LMCC group classified in the reference example and the β values of each CpG site in 34 cases in the HMCC group were performed. Furthermore, separately from the above 97 cases, β values of the CpG sites were also measured for the measured and calculated normal large intestine mucous membrane samples (n = 10), and further narrowing of the CpG sites focusing on the coincidence between the determination of methylation and the actual treatment outcome and the like were performed in consideration of the result. Then, CpG sites of the following bases at 16 sites were selected from 486,428 CpG sites targeted by the comprehensive DNA methylation analysis. As a result of the selection, it is considered that it is possible to determine whether the subject is in the LMCC group or the HMCC group, that is, whether or not the subject has sensitivity of chemotherapy against colorectal cancer, by using methylation of some or all of the bases at 16 sites described in the following Table 1 as an indicator.

[Table 1]

| Target ID | Position information |
|---|---|
| cg07319626 | Base at position 207308244 of chromosome 2 |
| cg02610058 | Base at position 241758901 of chromosome 2 |
| cg13803214 | Base at position 150804490 of chromosome 3 |

(continued)

| Target ID | Position information |
|---|---|
| cg14235416 | Base at position 141348307 of chromosome 4 |
| cg24642320 | Base at position 186049687 of chromosome 4 |
| cg25203704 | Base at position 17216922 of chromosome 5 |
| cg26129310 | Base at position 37664451 of chromosome 6 |
| cg18960642 | Base at position 39281694 of chromosome 6 |
| cg07413609 | Base at position 42276816 of chromosome 7 |
| cg22738219 | Base at position 13043313 of chromosome 10 |
| cg20629468 | Base at position 81664583 of chromosome 10 |
| cg20649951 | Base at position 88126306 of chromosome 10 |
| cg25303599 | Base at position 61595807 of chromosome 11 |
| cg01557297 | Base at position 23822017 of chromosome 14 |
| cg12379948 | Base at position 44896424 of chromosome 17 |
| cg14730085 | Base at position 39522548 of chromosome 19 |

Example 2

Among 83 cases of advanced and recurrent colorectal cancer (cases of RAS gene mutant type n = 18, cases of RAS gene wild type n = 65) having a history of treatment with an anti-EGFR antibody drug collected backward, 65 cases of RAS gene wild type were subjected to measurement of DNA methylation status by real-time PCR according to the following procedure, and classified into either an HMCC group or an LMCC group.

Determination of methylation using real-time PCR

The DNA sample of each case was subjected to bisulfite treatment under the following conditions, and then ΔCt value was determined by real-time PCR:

- Bisulfite treatment

- Bisulfite kit: EZ DNA Methylation Gold Kit
- Amount of DNA: 500 to 600 ng/rxn

  - Reagent configuration of methylation detection system

- Amount of DNA used for qPCR: 10 to 50 ng/Well
- Enzyme used: GoTaqG2 Hot Start Master Mix (final concentration of $MgCl_2$: 2.0 mM)
- Target: Primer final concentration 1000 nM/Probe (FAM) final concentration 500 nM
- Control: Primer final concentration 500 nM/Probe (HEX) final concentration 250 nM
- ACTB Promoter region (GenBank Y00474.1)

- Reaction temperature conditions
  95°C: 2 min→(95°C 15 sec→55°C 30 sec→72°C 1 min) x 50 cycles
- Measuring instrument: Bio-Rad CFX96

Target primer-probe sets and control primer-probe sets used for real-time PCR are as follows:

[Table 2]

| Probe ID | Chromosome No. | Base position of amplification range | Primer/probe |
|---|---|---|---|
| 1 | 2 | Positions 207308134 to 207308267 | Primer set 1, probe 1 (SEQ ID NOs: 1, 2, 3) |
| 2 | 2 | Positions 241758885 to 241759011 | Primer set 2, probe 2 (SEQ ID NOs: 4, 5, 6) |
| 3 | 3 | Positions 150804402 to 150804508 | Primer set 3, probe 3 (SEQ ID NOs: 7, 8, 9) |
| 4 | 4 | Positions 141348215 to 141348329 | Primer set 4, probe 4 (SEQ ID NOs: 10, 11, 12) |
| 5 | 4 | Positions 186049616 to 186049705 | Primer set 5, probe 5 (SEQ ID NOs: 13, 14, 15) |
| 6 | 5 | Positions 17216901 to 17217025 | Primer set 6, probe 6 (SEQ ID NOs: 16, 17, 18) |
| 7 | 6 | Positions 37664344 to 37664472 | Primer set 7, probe 7 (SEQ ID NOs: 19, 20, 21) |
| 8 | 6 | Positions 39281671 to 39281814 | Primer set 8, probe 8 (SEQ ID NOs: 22, 23, 24) |
| 9 | 7 | Positions 42276765 to 42276874 | Primer set 9, probe 9 (SEQ ID NOs: 25, 26, 27) |
| 10 | 10 | Positions 13043227 to 13043334 | Primer set 10, probe 10 (SEQ ID NOs: 28,29, 30) |
| 11 | 10 | Positions 81664566 to 81664662 | Primer set 11, probe 11 (SEQ ID NOs: 31, 32, 33) |
| 12 | 10 | Positions 88126244 to 88126334 | Primer set 12, probe 12 (SEQ ID NOs: 34, 35, 36) |
| 13 | 11 | Positions 61595787 to 61595864 | Primer set 13, probe 13 (SEQ ID NOs: 37, 38, 39) |
| 14 | 14 | Positions 23821996 to 23822092 | Primer set 14, probe 14 (SEQ ID NOs: 40, 41, 42) |
| 15 | 17 | Positions 44896309 to 44896450 | Primer set 15, probe 15 (SEQ ID NOs: 43, 44, 45) |

(continued)

| Probe ID | Chromosome No. | Base position of amplification range | Primer/probe |
|---|---|---|---|
| 16 | 19 | Positions 39522494 to 39522608 | Primer set 16, probe 16 (SEQ ID NOs: 46, 47, 48) |
| Control (common to each region) | | | Primer set:<br>GGAGGTAGGGAGTATATAGGTTG (SEQ ID NO: 49)<br>ACTCCTCCCTTAAAAATTACAAAAA (SEQ ID NO: 50)<br>Probe:<br>CCACCACCCAACACACAATAACA (SEQ ID NO: 51) |

When the ΔCt value was less than 4 as a result of real-time PCR using the 16 analysis region primer-probe sets and 1 control primer-probe set, methylation of the analysis region was determined to be positive. Then, a case in which the number of methylation-positive regions was 8 or more among the 16 analysis regions was defined as an HMCC group, and a case in which the number of methylation-positive regions was less than 8 was defined as an LMCC group. In addition, treatment outcome (progression-free survival: PFS and response rate) with an anti-EGFR antibody drug (cetuximab or panitumumab) was compared and examined for each group.

First, the ratios of the RAS gene mutant type group, the HMCC group and the LMCC group among the 83 cases are shown in the following Table 3.

[Table 3]

| *RAS* gene type | Mutant type (n = 18) | Wild type (n = 65) | |
|---|---|---|---|
| Methylation status | | HMCC | LMCC |
| n (%) | 18 (21.7) | 16 (19.3) | 49 (59.0) |

In addition, the response rate of each group is shown in the following Table 4.

[Table 4]

| *RAS* gene type | Mutant type (n = 18) | Wild type (n = 65) | |
|---|---|---|---|
| Methylation status | | HMCC | LMCC |
| n (%) | 1/18 (5.6) | 1/16 (6.3) | 18/49 (39.1) |

Further, a graph summarizing progression-free survival: PFS for each group is shown in Fig. 1. As can be seen from these results, by measuring the DNA methylation status, an anti-EGFR antibody drug resistant group in RAS wild-type advanced and recurrent colorectal cancer could be extracted.

Example 3

It is known that prognosis of advanced colorectal cancer varies depending on location of primary lesion. Therefore, 65 cases of advanced and recurrent colorectal cancer in Example 2 were further classified by the location of primary lesion (right colon and left large intestine), and it was examined which classification method was more excellent in therapeutic effect prediction accuracy. First, the response rates of HMCC and LMCC groups and the response rates for each location of the primary lesion are shown in the following Table 5.

[Table 5]

| Methylation status | n (%) |
|---|---|
| HMCC group (n = 16) | 1 (6.3%) |
| LMCC group (n = 49) | 18 (39.1%) |

| Classification by primary lesion | n (%) |
|---|---|
| Right colon (n = 23) | 4 (19.0%) |
| Left large intestine (n = 42) | 15 (36.6%) |

Furthermore, Fig. 2 shows a graph summarizing progression-free survival: PFS for each of the HMCC group (L-HMCC group) in which the primary lesion was in the left large intestine, the HMCC group (R-HMCC group) in which the primary lesion was in the right colon, the LMCC group (L-LMCC group) in which the primary lesion was in the left large intestine, and the LMCC group (R-LMCC group) in which the primary lesion was in the right colon. As shown in Fig. 2, there were cases of the HMCC group and the LMCC group among cases where the primary lesion was in the left large intestine, and there was a statistically significant difference in the median PFS between both groups. In addition, there were cases of the HMCC group and the LMCC group among cases where the primary lesion was in the right colon, and there was a statistically significant difference in the median PFS between both groups. Therefore, it can be seen that the determination using methylation as an indicator is useful as an indicator different from the location of the primary lesion. In addition, Table 6 shows results of analyzing difference in PFS for each of the location of the primary lesion, the RAS gene mutation, and the methylation status.

[Table 6]

| | Progression-free survival | | | |
|---|---|---|---|---|
| | Univariate analysis | | Multivariate analysis | |
| Parameter | Hazard ratio (95% confidence interval) | p Value | Hazard ratio (95% confidence interval) | p Value |
| Primary lesion (right large intestine vs. left large intestine) | 0.66 (0.41-1.09) | 0.10 | 0.92 (0.55-1.57) | 0.75 |
| RAS gene (wild type vs. mutant type) | 0.43 (0.25-0.79) | < 0.01 | 0.41 (0.24-0.76) | < 0.01 |
| Methylation status (LMCC vs. HMCC) | 0.37 (0.21-0.67) | < 0.01 | 0.36 (0.20-0.69) | < 0.01 |

As shown in Table 6, since a hazard ratio of the methylation status was the smallest, it was suggested that among these indicators, a method using methylation as an indicator was excellent in therapeutic effect prediction accuracy.

Example 4

The ΔCt value was determined by real-time PCR in the same manner as in Example 2 except that 83 cases of advanced and recurrent colorectal cancer in which the above-described KRAS gene is wild type were measured and only the probe ID1 targeting a region containing cg07319626 was used (thus, primer set 1 and probe 1 were used). As a result of the real-time PCR, a case in which the ΔCt value was less than 4 was defined as the HMCC group, and a case in which the ΔCt value was 4 or more was defined as the LMCC group. In addition, treatment outcome (progression-free survival: PFS and response rate) with an anti-EGFR antibody drug (cetuximab or panitumumab) was compared and examined for each group.

A graph summarizing progression-free survival: PFS for each group is shown in Fig. 3. In addition, Fig. 4 shows a graph summarizing PFS for the HMCC group and the LMCC group for 65 cases in which the RAS gene is wild type among 83 cases of advanced and recurrent colorectal cancer in which the KRAS gene is wild type. As can be seen from these results, by using methylation of only one region as an indicator, an anti-EGFR antibody drug resistant group in advanced and recurrent colorectal cancer could be extracted.

Example 5

Figs. 5 to 20 show graphs showing β values calculated by comprehensive DNA methylation analysis using Infinium 450K (Illumina) of surrounding CpG sites, at each of the 16 CpG sites used as indicators in the Examples. In each drawing, each row shows a respective case. Also, in the drawing, "Target ID" is an ID of a CpG site, "CHR" is a chromosome number, and "MAPINFO" indicates which base on the chromosome is methylated. Moreover, in each drawing, a column with a β value of less than 0.4 is shown in dark gray, a column with a β value of more than 0.3 and 0.4 or less is shown in light gray, and a column with a β value of 0.3 or less is shown in white.

As shown in Figs. 5 to 20, the β value tends to be larger in the HMCC cases than in the LMCC cases also in CpG sites before and after the 16 CpG sites used as indicators in Example 1. Therefore, for example, it is considered that by using methylation of one region (for example, a region at positions 207307150 to 207309004 of chromosome 2 (region (i)) shown in Fig. 5) among 16 regions shown in Figs. 5 to 20 as an indicator, it is possible to determine whether the subject is in either the LMCC group or the HMCC group, and thus whether or not the subject has sensitivity of chemotherapy against colorectal cancer. For example, using TargetID cg24399540, TargetID cg27380819, TargetID cg04307977, TargetID cg06123396, TargetID cg09454676, TargetID cg08804846, TargetID cg26258845, TargetID cg07319626, TargetID cg16778809, TargetID cg09553380, and TargetID cg21823149 as indicators in Fig. 5, when a CpG site is defined as a highly methylated region when the β value is 0.4 or more and it is determined as HMCC when the number of highly methylated regions is 8 or more, the HMCC group and the LMCC group can be classified with significant difference in the median PFS, 82 days and 224 days ($p = < 0.001$), respectively.

## Claims

1. A method for testing for sensitivity of chemotherapy against colorectal cancer, the method using, as an indicator, methylation in at least one site selected from the group consisting of CpG sites comprised in the following regions (i) to (xvi) in DNA collected from a colorectal cancer patient:

   Region (i): a region at positions 207307150 to 207309004 of chromosome 2
   Region (ii): a region at positions 241758282 to 241760510 of chromosome 2
   Region (iii): a region at positions 150802997 to 150805168 of chromosome 3
   Region (iv): a region at positions 141347993 to 141348489 of chromosome 4
   Region (v): a region at positions 186048714 to 186050048 of chromosome 4
   Region (vi): a region at positions 17216679 to 17219240 of chromosome 5
   Region (vii): a region at positions 37663982 to 37664539 of chromosome 6
   Region (viii): a region at positions 39281183 to 39282332 of chromosome 6
   Region (ix): a region at positions 42273390 to 42277951 of chromosome 7
   Region (x): a region at positions 13039715 to 13043422 of chromosome 10
   Region (xi): a region at positions 81664401 to 81665076 of chromosome 10
   Region (xii): a region at positions 88126287 to 88127189 of chromosome 10
   Region (xiii): a region at positions 61595807 to 61596627 of chromosome 11
   Region (xiv): a region at positions 23820337 to 23822266 of chromosome 14
   Region (xv): a region at positions 44895317 to 44896749 of chromosome 17
   Region (xvi): a region at positions 39520228 to 39523159 of chromosome 19.

2. The method according to claim 1, comprising detecting methylation in at least one site selected from the group consisting of bases at:

   positions 207307150, 207307490, 207307544, 207307622, 207307732, 207308004, 207308087, 207308244, 207308375, 207308829, 207309003, 241758282, 241758399, 241758805, 241758901, 241759279, 241759414, 241760025, 241760164, 241760190, and 241760509 of chromosome 2,
   positions 150802997, 150803295, 150803307, 150803666, 150803669, 150804058, 150804063, 150804313, 150804490, 150804696, 150804719, and 150805167 of chromosome 3,
   positions 141347993, 141348043, 141348167, 141348307, 141348488, 186048714, 186048907, 186049687, 186049926, and 186050047 of chromosome 4, positions 17216679, 17216922, 17217093, 17217877, 17218089, 17218278, 17218308, 17218547, 17218778, 17219020, 17219128, 17219226, and 17219239 of chromosome 5,
   positions 37663982, 37664451, 37664538, 39281183, 39281421, 39281450, 39281541, 39281694, 39281885, 39282164, 39282316, and 39282331 of chromosome 6,
   positions 42273390, 42275601, 42275813, 42275870, 42275872, 42276004, 42276814, 42276816, 42276819, 42276848, 42276881, 42276890, 42276941, 42276981, 42277044, 42277066, 42277071, 42277347, 42277375, 42277394, 42277410, 42277807, and 42277950 of chromosome 7,
   positions 13039715, 13041989, 13043313, 13043421, 81664401, 81664567, 81664583, 81664698, 81664955, 81665075, 88126287, 88126291, 88126299, 88126306, 88126853, and 88127188 of chromosome 10,
   positions 61595807, 61595956, 61596068, 61596307, 61596333, 61596405, and 61596626 of chromosome 11,
   positions 23820337, 23821149, 23821229, 23821435, 23821445, 23821570, 23821596, 23821902, 23822017, and 23822265 of chromosome 14,
   positions 44895317, 44896017, 44896080, 44896147, 44896162, 44896166, 44896168, 44896212, 44896223, 44896424, and 44896748 of chromosome 17, and
   positions 39520228, 39521931, 39522418, 39522548, 39522747, 39522944, 39523083, and 39523158 of chromosome 19, and using the detection result as an indicator of sensitivity of chemotherapy against colorectal cancer.

3. The method according to claim 1 or 2, wherein methylation in at least one site selected from the group consisting of CpG sites comprised in the following regions (i') to (iv'), regions (viii') to (x'), and regions (xii') to (xvi') is used as an indicator:

   Region (i'): a region at positions 207307490 to 207308376 of chromosome 2
   Region (ii') a region at positions 241758805 to 241760510 of chromosome 2
   Region (iii'): a region at positions 150802997 to 150804720 of chromosome 3

Region (iv'): a region at positions 141348167 to 141348308 of chromosome 4
Region (viii'): a region at positions 39281541 to 39282165 of chromosome 6
Region (ix'): a region at positions 42275870 to 42276817 of chromosome 7
Region (x'): a region at positions 13041989 to 13043422 of chromosome 10
Region (xii'): a region at positions 88126287 to 88126307 of chromosome 10
Region (xiii'): a region at positions 61595807 to 61596334 of chromosome 11
Region (xiv'): a region at positions 23821596 to 23822266 of chromosome 14
Region (xv'): a region at positions 44895317 to 44896425 of chromosome 17
Region (xvi'): a region at positions 39520228 to 39523084 of chromosome 19.

**4.** The method according to claim 1, comprising detecting methylation in at least one site selected from the group consisting of bases at:

positions 207308141, 207308149, 207308153, 207308172, 207308177, 207308181, 207308185, 207308244, 207308247, 207308267, 241758885, 241758889, 241758896, 241758901, 241758919, 241758922, 241758931, 241758936, 241758940, 241759003, 241759005, and 241759009 of chromosome 2,
positions 150804417, 150804420, 150804474, 150804479, 150804486, 150804490, 150804496, 150804504, and 150804507 of chromosome 3,
positions 141348224, 141348232, 141348272, 141348282, 141348289, 141348307, 141348318, 141348324, 186049623, 186049636, 186049659, 186049670, 186049685, 186049687, 186049689, and 186049705 of chromosome 4,
positions 17216904, 17216916, 17216922, 17216940, 17216949, 17216952, 17217003, 17217011, 17217017, and 17217023 of chromosome 5,
positions 37664352, 37664366, 37664419, 37664424, 37664451, 37664460, 39281692, 39281694, 39281723, 39281729, 39281735, 39281738, 39281797, 39281806, 39281808, and 39281813 of chromosome 6,
positions 42276764, 42276767, 42276769, 42276783, 42276810, 42276814, 42276816, 42276819, 42276825, 42276852, 42276862, and 42276874 of chromosome 7,
positions 13043231, 13043247, 13043285, 13043288, 13043313, 13043321, 13043333, 81664567, 81664573, 81664583, 81664598, 81664600, 81664606, 81664614, 81664636, 81664656, 88126243, 88126250, 88126259, 88126285, 88126287, 88126291, 88126299, 88126306, and 88126310 of chromosome 10,
positions 61595796, 61595807, 61595815, 61595818, 61595820, 61595829, 61595843, and 61595852 of chromosome 11,
positions 23822015, 23822017, 23822043, 23822054, 23822073, 23822075, and 23822079 of chromosome 14,
positions 44896314, 44896316, 44896324, 44896386, 44896390, 44896401, 44896424, 44896443, and 44896450 of chromosome 17, and
positions 39522493, 39522510, 39522533, 39522537, 39522548, 39522550, 39522552, 39522582, 39522587, 39522591, and 39522608 of chromosome 19, and using the detection result as an indicator of sensitivity of chemotherapy against colorectal cancer.

**5.** The method according to any one of claims 1 to 4, wherein methylation in at least one site selected from the group consisting of CpG sites comprised in the following regions is used as an indicator:

a region at positions 207308134 to 207308268 of chromosome 2
a region at positions 241758885 to 241759011 of chromosome 2
a region at positions 150804402 to 150804508 of chromosome 3
a region at positions 141348215 to 141348329 of chromosome 4
a region at positions 186049616 to 186049706 of chromosome 4
a region at positions 17216901 to 17217025 of chromosome 5
a region at positions 37664344 to 37664472 of chromosome 6
a region at positions 39281671 to 39281814 of chromosome 6
a region at positions 42276764 to 42276875 of chromosome 7
a region at positions 13043227 to 13043334 of chromosome 10
a region at positions 81664566 to 81664662 of chromosome 10
a region at positions 88126243 to 88126334 of chromosome 10
a region at positions 61595787 to 61595864 of chromosome 11
a region at positions 23821996 to 23822092 of chromosome 14
a region at positions 44896309 to 44896451 of chromosome 17
a region at positions 39522493 to 39522609 of chromosome 19.

**6.** The method according to any one of claims 1 to 5, wherein the CpG site is located in a region to which an oligonucleotide of at least one sequence selected from the group consisting of SEQ ID NOs: 1 to 48 hybridizes, in DNA obtained by bisulfite treatment of DNA containing the CpG site that is methylated.

**7.** The method according to any one of claims 1 to 6, comprising the steps of:

(1) detecting a plurality of CpG sites comprised in each of at least two regions among the regions (i) to (xvi) and measuring frequency of methylation of the detected CpG sites,
(2) in said each region detected in the step (1), when the frequency of methylation in the region was equal to or more than a preset value, determining the region as a highly methylated region, and
(3) in said each region measured in the step (1), when a ratio of the regions determined as the high methylated region in the step (2) is equal to or more than a preset ratio, determining that there is no sensitivity of chemotherapy against colorectal cancer.

**8.** The method according to any one of claims 1 to 6, comprising the steps of:

(1) detecting a plurality of CpG sites comprised in each of at least two regions among the regions (i) to (xvi) and measuring frequency of methylation of the detected CpG sites,
(2) in said each region measured in the step (1), when the frequency of methylation in the region was equal to or more than a preset value, determining the region as a highly methylated region, and
(3) in said each region measured in the step (1), when a ratio of the regions determined as the high methylated region in the step (2) is less than a preset ratio, determining that there is sensitivity of chemotherapy against colorectal cancer.

**9.** The method according to claim 7 or 8, wherein the frequency of methylation in each region in the step (1) is measured by real-time PCR, and when a $\Delta$Ct value that is a difference between a Ct value measured by real-time PCR and a Ct value of a control reaction is less than a preset value, the region is defined as the highly methylated region.

**10.** The method according to any one of claims 7 to 9, wherein the frequency of methylation is measured for at least 8 regions among the regions (i) to (xvi) in the step (1).

**11.** The method according to any one of claims 1 to 10, wherein the DNA collected from the colorectal cancer patient is DNA prepared from colorectal tissue, blood, serum, plasma, feces, intestinal lavage solution or enema lavage solution collected from the colorectal cancer patient.

**12.** The method according to any one of claims 1 to 11, wherein the chemotherapy against colorectal cancer is a chemotherapy using an anti-EGFR antibody.

**13.** The method according to claim 12, wherein the anti-EGFR antibody is at least one selected from the group consisting of cetuximab and panitumumab.

**14.** A kit for acquiring information that can be an indicator of presence or absence of sensitivity of chemotherapy against colorectal cancer, the kit comprising a primer set for analyzing methylation in at least one site selected from the group consisting of CpG sites comprised in the following regions (i) to (xvi) in DNA collected from a colorectal cancer patient:

Region (i): a region at positions 207307150 to 207309004 of chromosome 2
Region (ii): a region at positions 241758282 to 241760510 of chromosome 2
Region (iii): a region at positions 150802997 to 150805168 of chromosome 3
Region (iv): a region at positions 141347993 to 141348489 of chromosome 4
Region (v): a region at positions 186048714 to 186050048 of chromosome 4
Region (vi): a region at positions 17216679 to 17219240 of chromosome 5
Region (vii): a region at positions 37663982 to 37664539 of chromosome 6
Region (viii): a region at positions 39281183 to 39282332 of chromosome 6
Region (ix): a region at positions 42273390 to 42277951 of chromosome 7
Region (x): a region at positions 13039715 to 13043422 of chromosome 10
Region (xi): a region at positions 81664401 to 81665076 of chromosome 10
Region (xii): a region at positions 88126287 to 88127189 of chromosome 10

Region (xiii): a region at positions 61595807 to 61596627 of chromosome 11
Region (xiv): a region at positions 23820337 to 23822266 of chromosome 14
Region (xv): a region at positions 44895317 to 44896749 of chromosome 17
Region (xvi): a region at positions 39520228 to 39523159 of chromosome 19.

**15.** The kit according to claim 14, wherein the primer set is a primer set for analyzing methylation of a CpG site by at least one method selected from the group consisting of a methylation-specific PCR method, a sequencing method, and a mass spectrometry method.

**16.** The kit according to claim 14 or 15, wherein the primer set comprises a primer set capable of amplifying at least one of the following regions after bisulfite treatment when a CpG site comprised in the region is methylated:

a region comprising positions 207308134 to 207308267 of chromosome 2
a region comprising positions 241758885 to 241759011 of chromosome 2
a region comprising positions 150804402 to 150804508 of chromosome 3
a region comprising positions 141348215 to 141348329 of chromosome 4
a region comprising positions 186049616 to 186049705 of chromosome 4
a region comprising positions 17216901 to 17217025 of chromosome 5
a region comprising positions 37664344 to 37664472 of chromosome 6
a region comprising positions 39281671 to 39281814 of chromosome 6
a region comprising positions 42276765 to 42276874 of chromosome 7
a region comprising positions 13043227 to 13043334 of chromosome 10
a region comprising positions 81664566 to 81664662 of chromosome 10
a region comprising positions 88126244 to 88126334 of chromosome 10
a region comprising positions 61595787 to 61595864 of chromosome 11
a region comprising positions 23821996 to 23822092 of chromosome 14
a region comprising positions 44896309 to 44896450 of chromosome 17
a region comprising positions 39522494 to 39522608 of chromosome 19.

FIG. 1

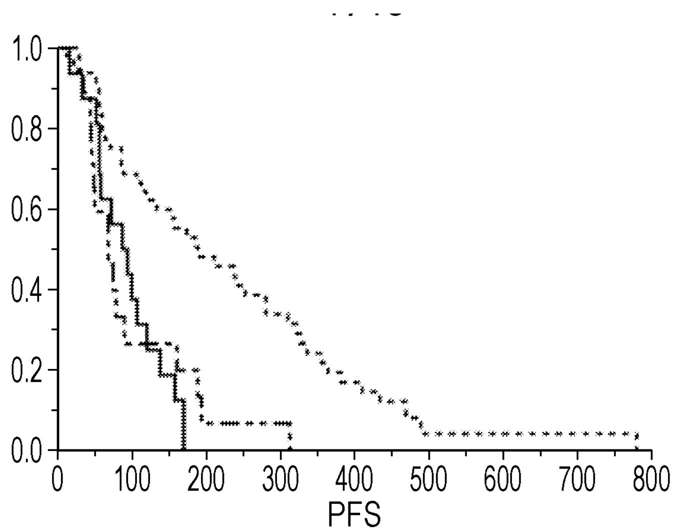


FIG. 2

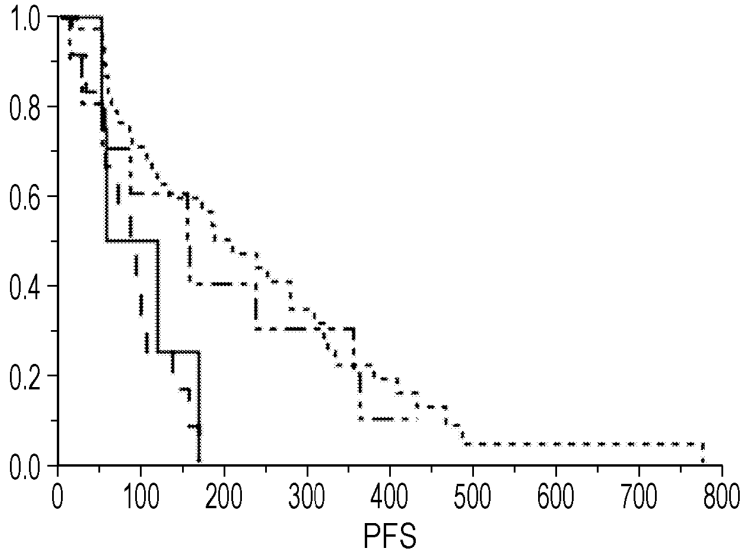


FIG. 3

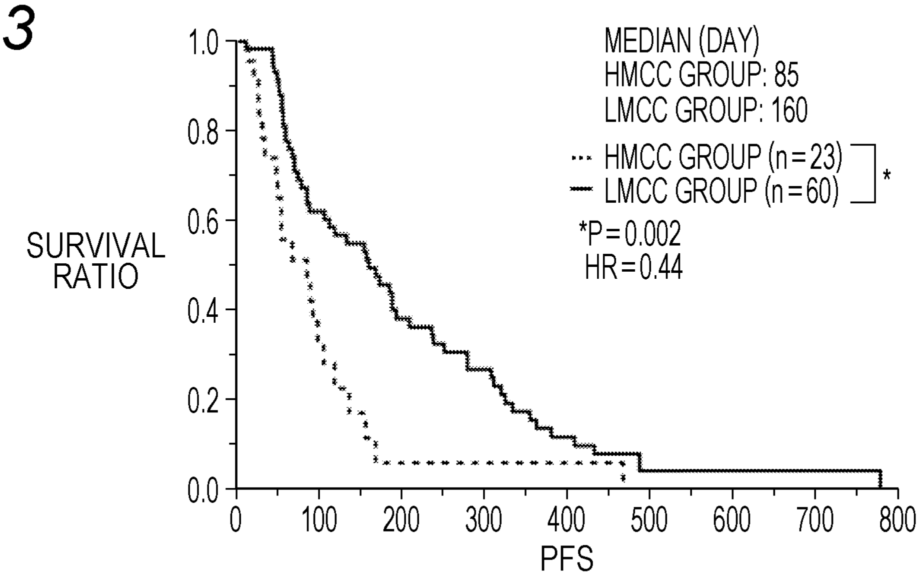

FIG. 4

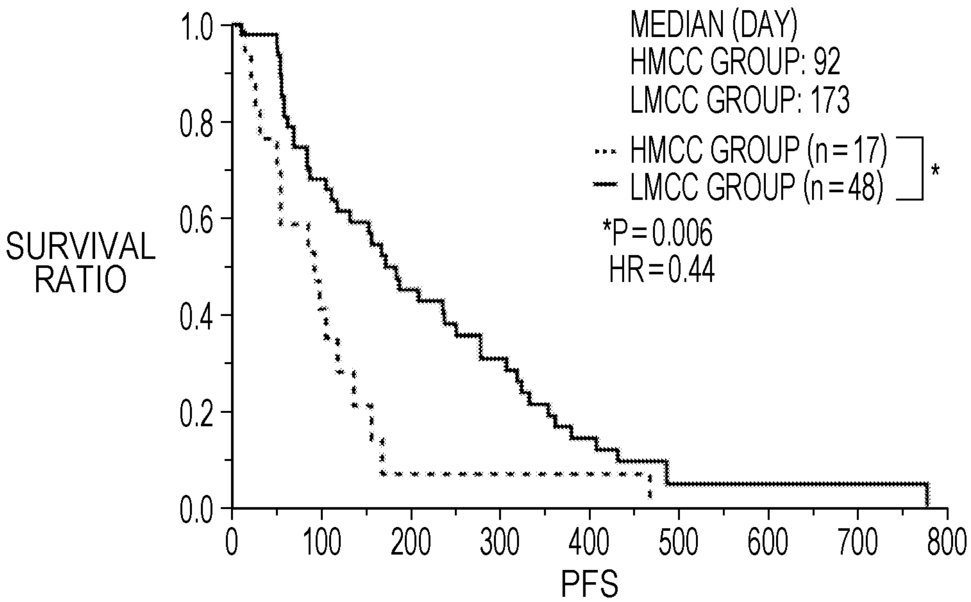
MEDIAN (DAY)
HMCC GROUP: 92
LMCC GROUP: 173
HMCC GROUP (n=17)
LMCC GROUP (n=48)
*
*P=0.006
HR=0.44
SURVIVAL RATIO
1.0
0.8
0.6
0.4
0.2
0.0
0
100
200
300
400
500
600
700
800
PFS

FIG. 5

REGION (i): TARGET PROBE cg07319626

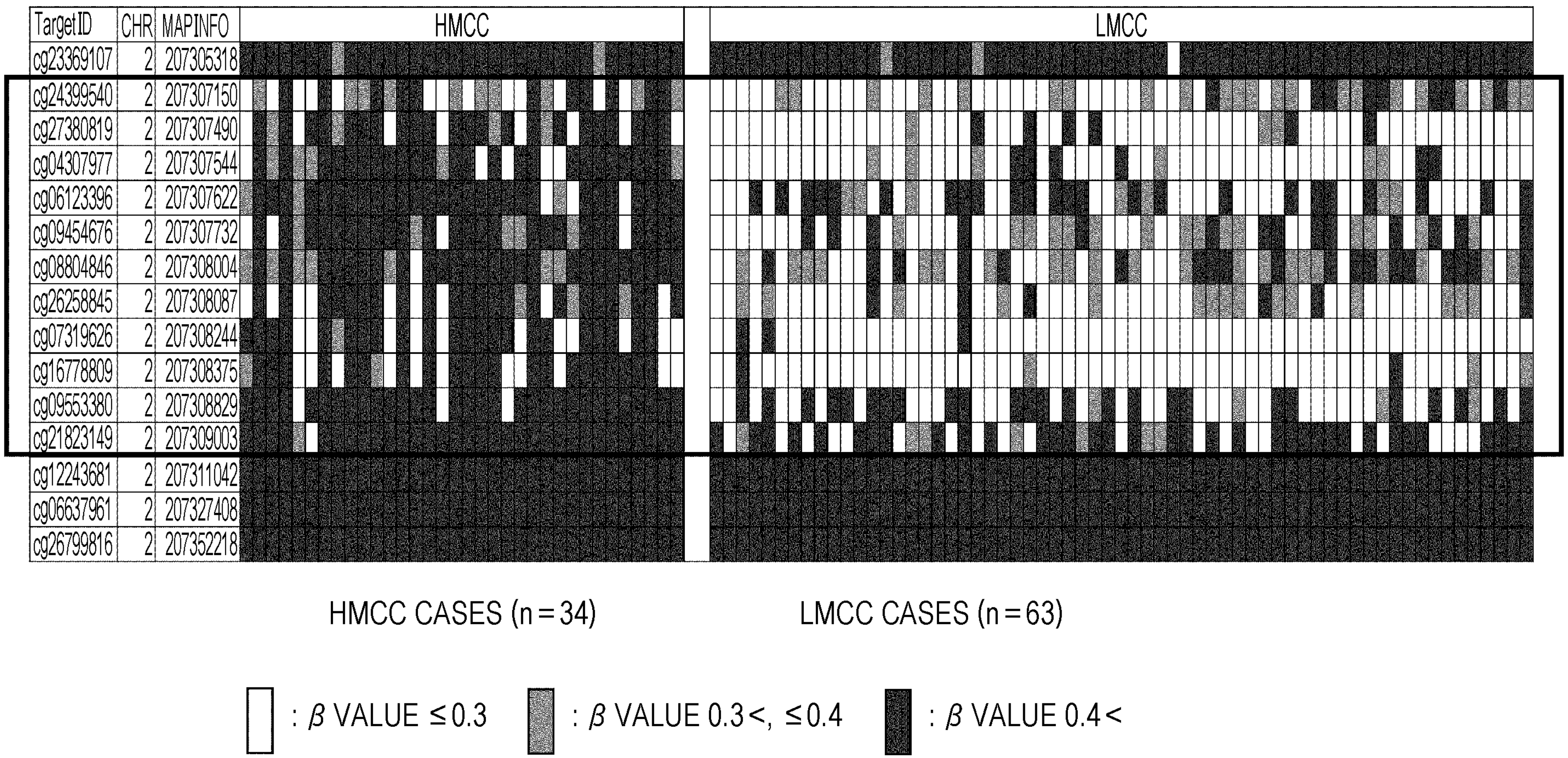

FIG. 6

REGION (ii): TARGET PROBE cg02610058

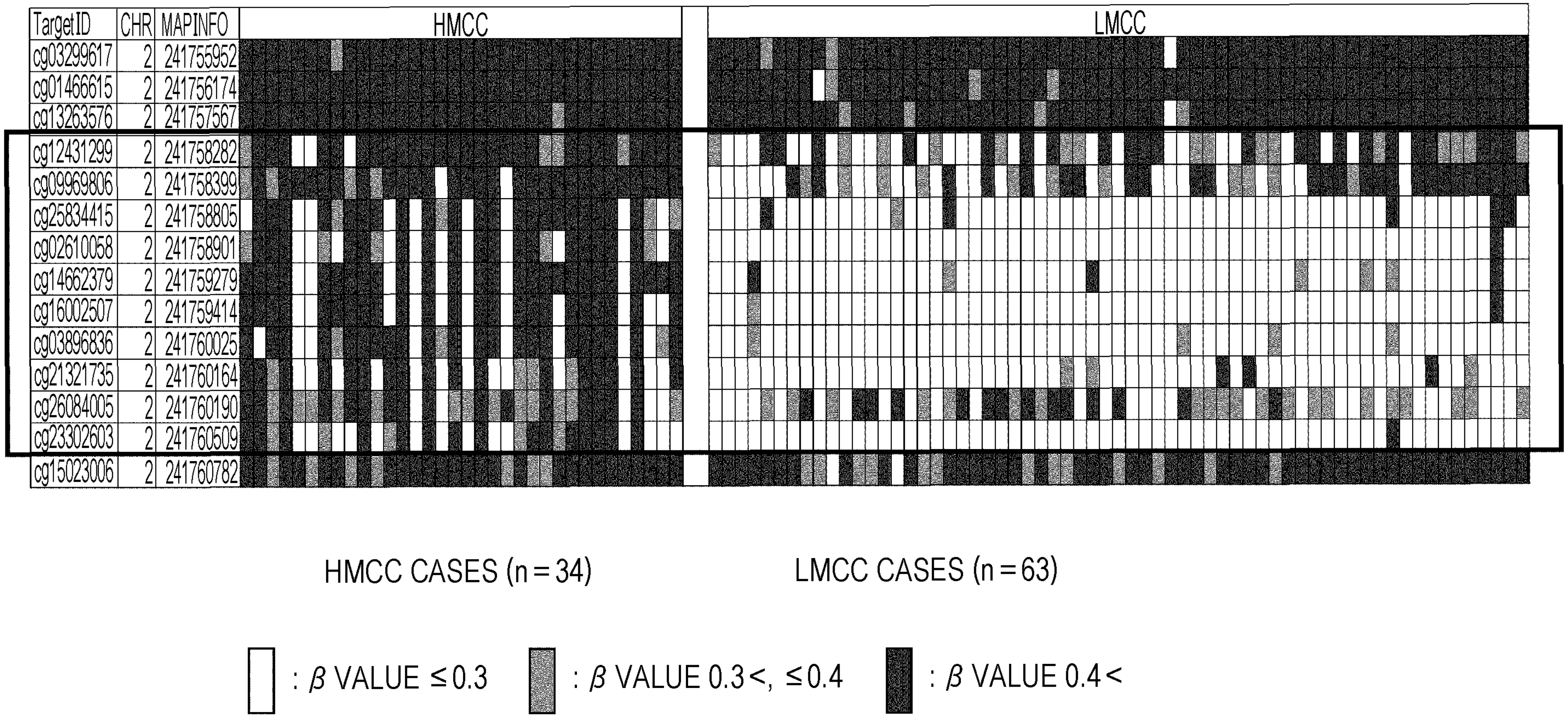

# FIG. 7

REGION (iii): TARGET PROBE cg13803214

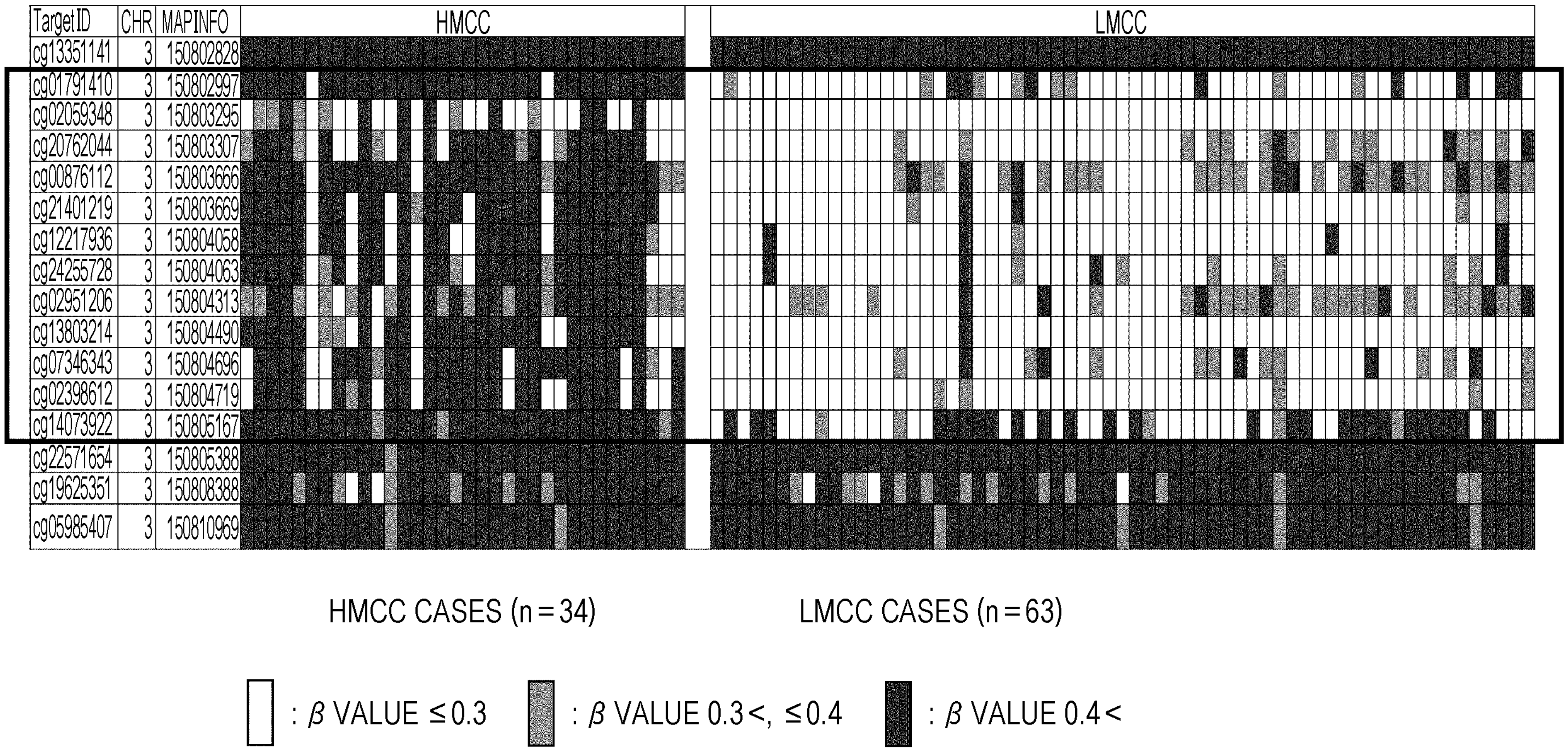

FIG. 8

REGION (iv): TARGET PROBE cg14235416

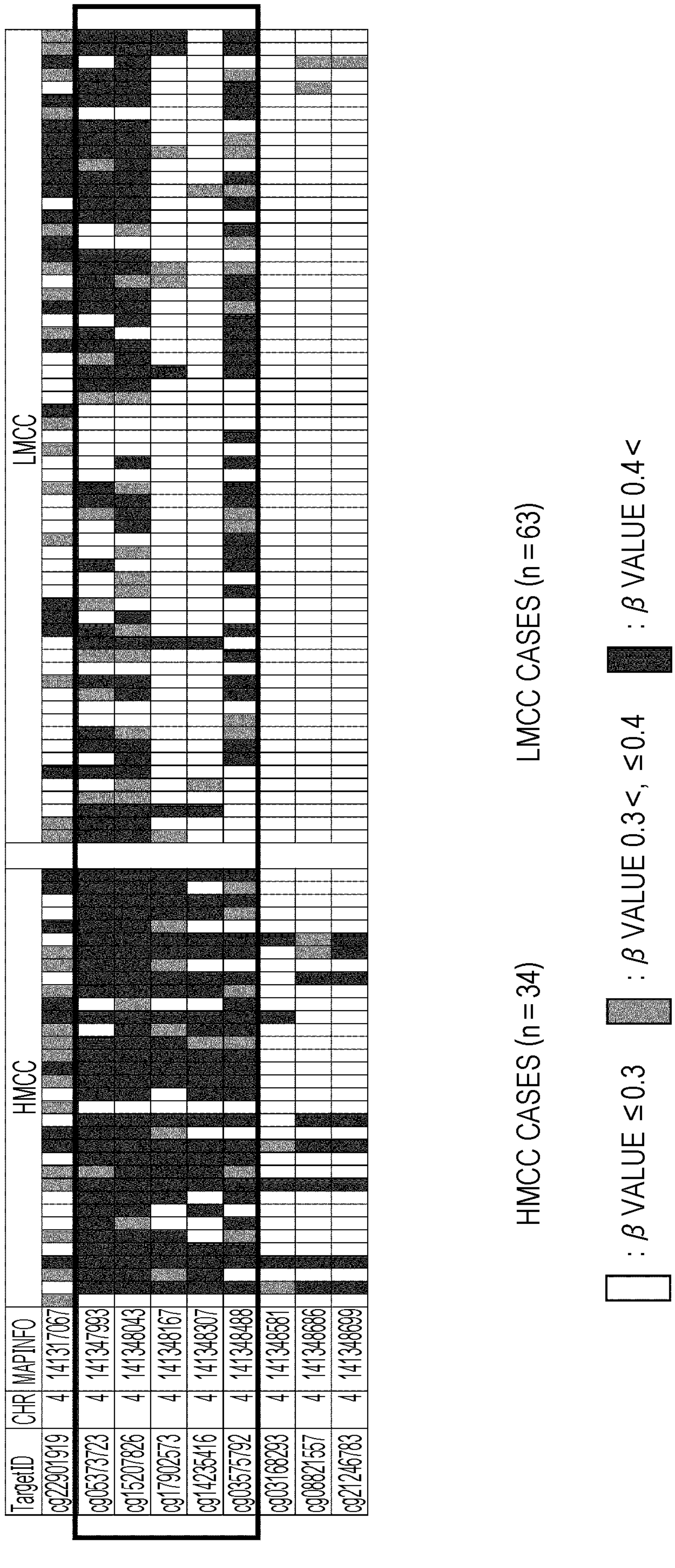

FIG. 9

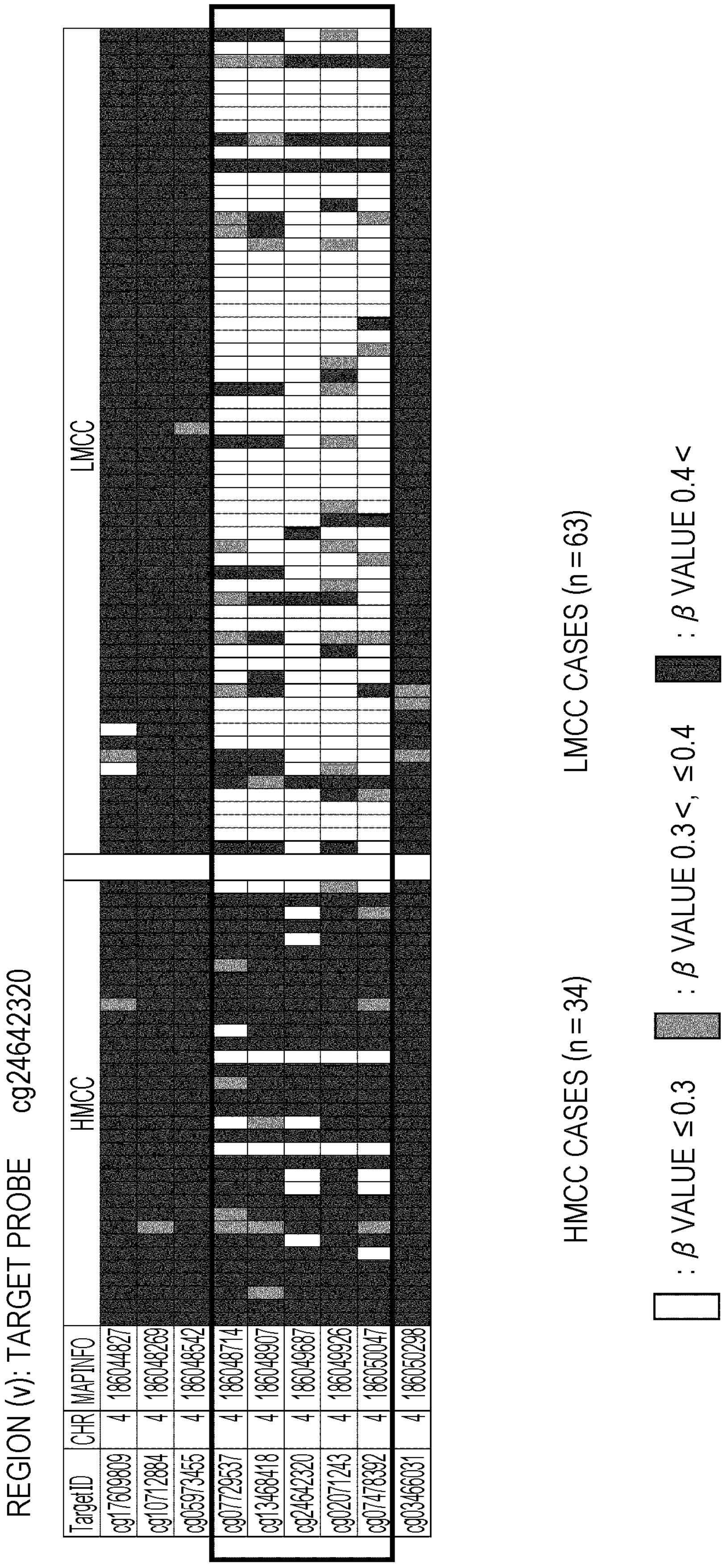
REGION (v): TARGET PROBE cg24642320
HMCC
LMCC
TargetID CHR MAPINFO
cg17609809 4 186044827
cg10712884 4 186048269
cg05973455 4 186048542
cg07729537 4 186048714
cg13468418 4 186048907
cg24642320 4 186049687
cg02071243 4 186049926
cg07478392 4 186050047
cg03466031 4 186050298
HMCC CASES (n=34)
LMCC CASES (n=63)
: β VALUE ≤0.3
: β VALUE 0.3<, ≤0.4
: β VALUE 0.4<

# FIG. 10

REGION (vi): TARGET PROBE cg25203704

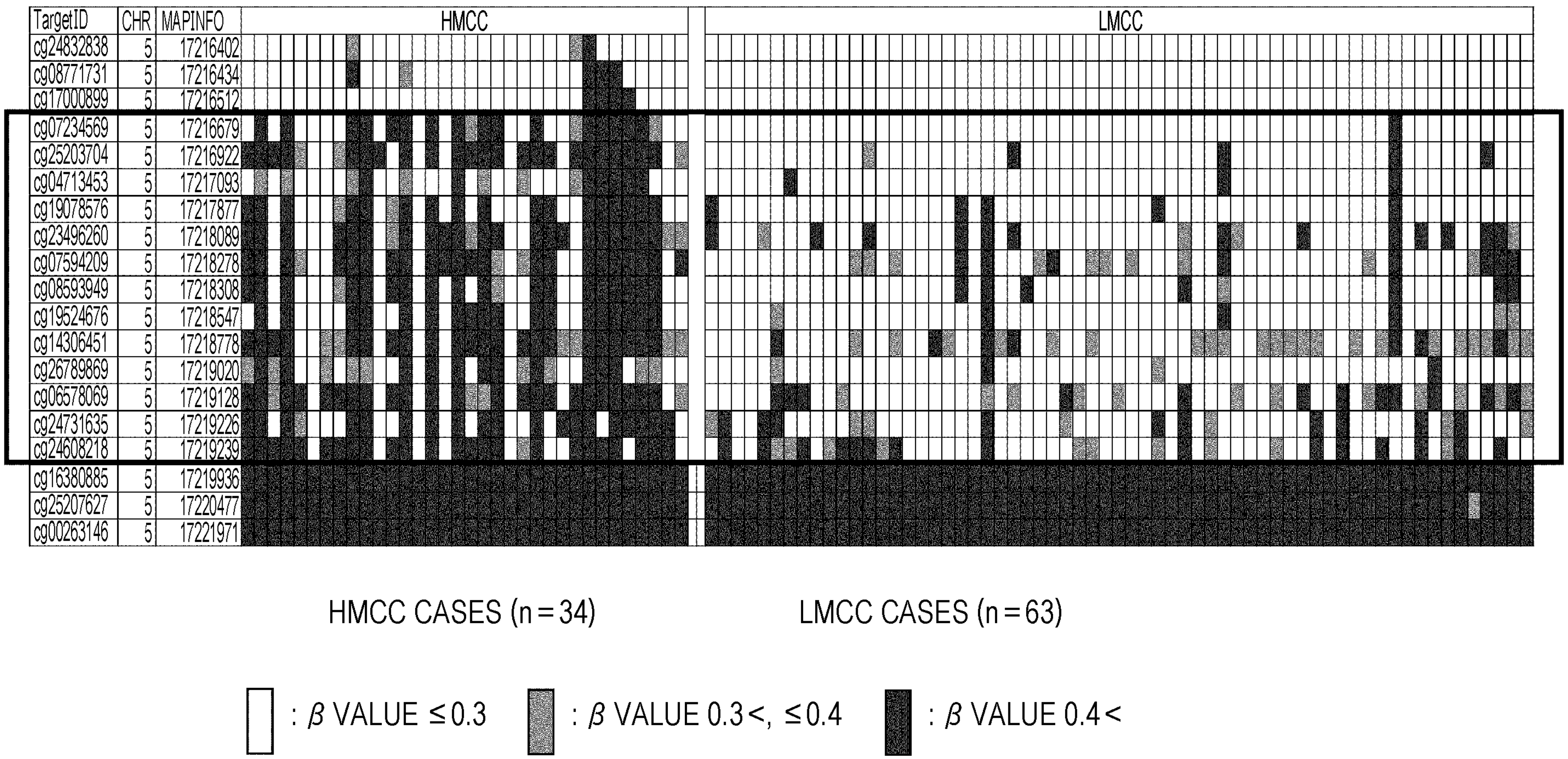

FIG. 11

REGION (vii): TARGET PROBE cg26129310

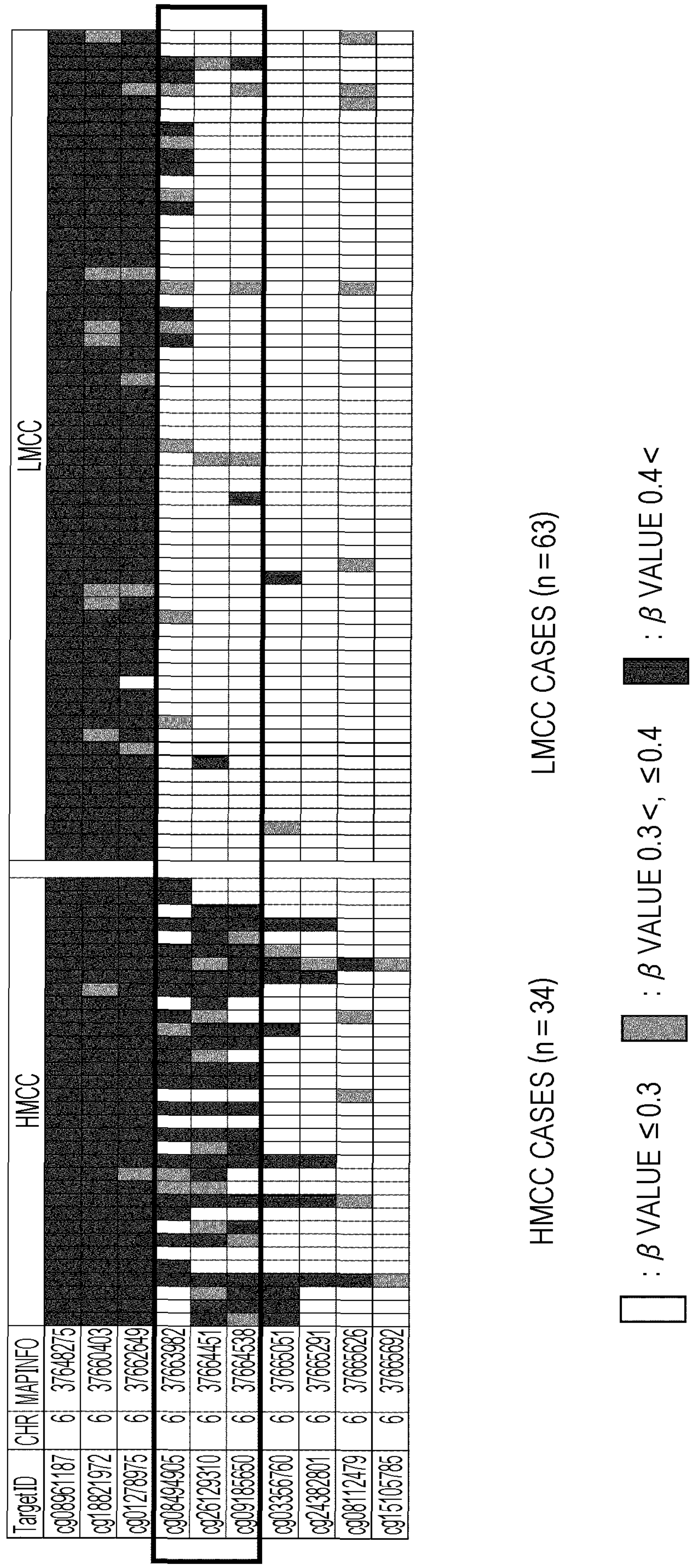

# FIG. 12

REGION (viii): TARGET PROBE cg18960642

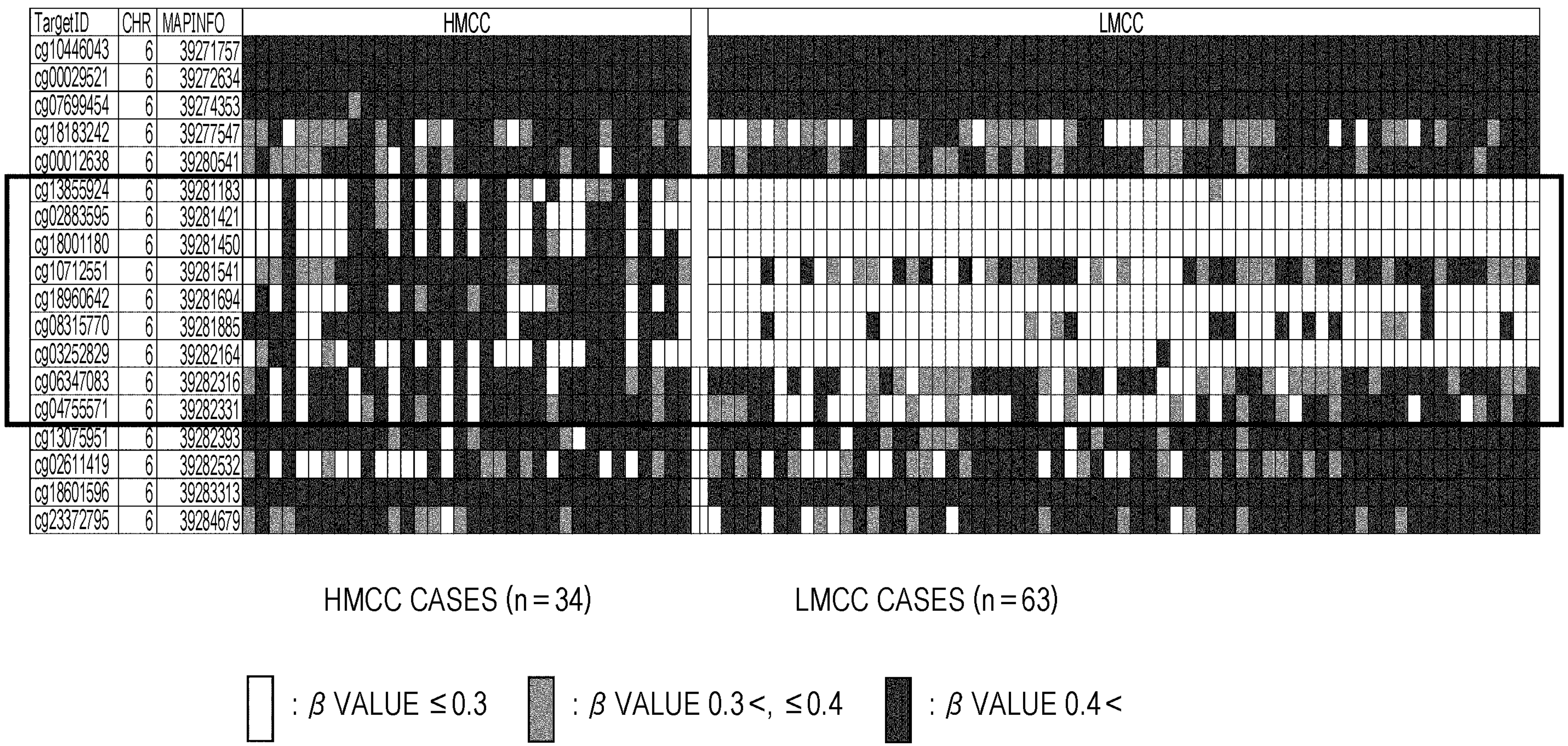

# FIG. 13

REGION (ix): TARGET PROBE cg07413609

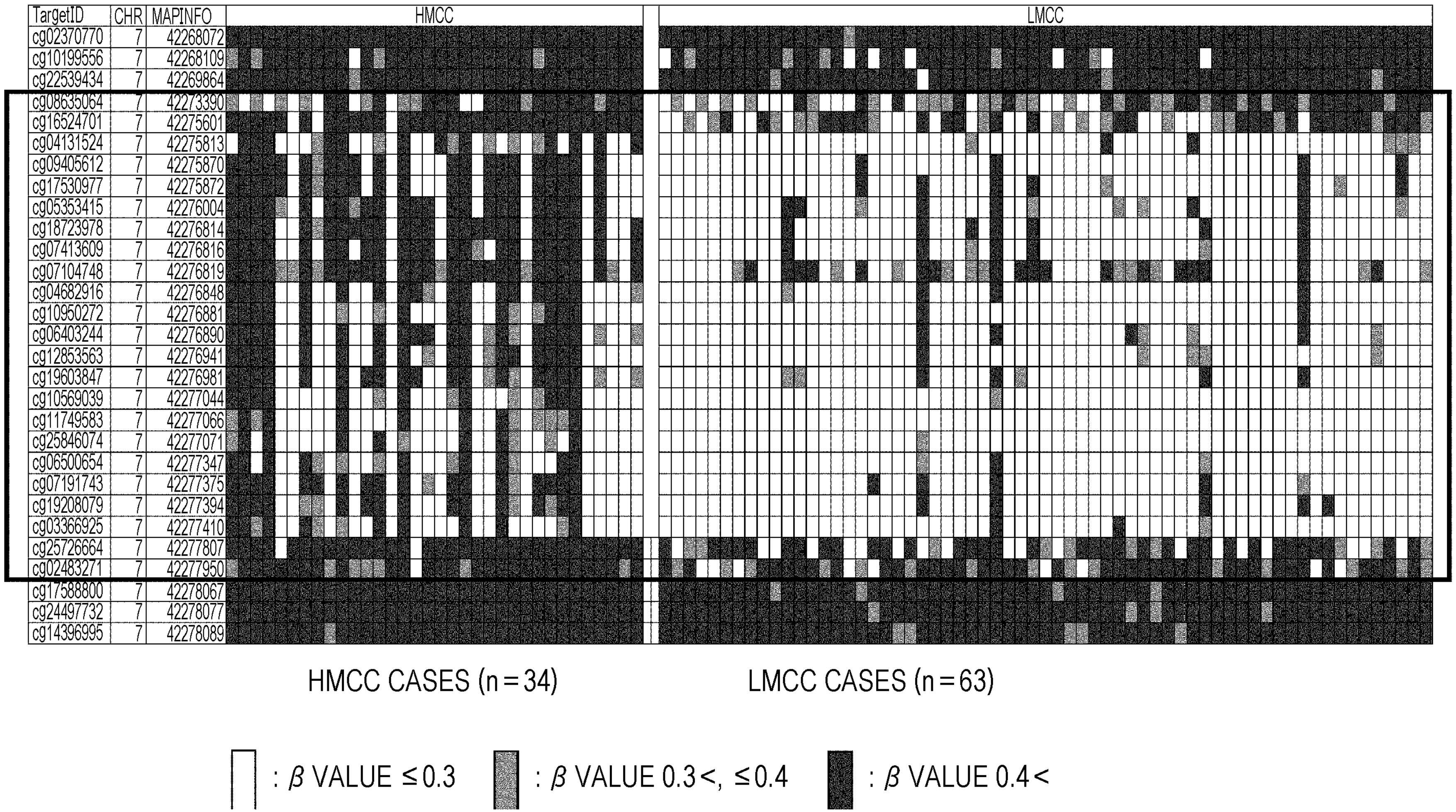

FIG. 14

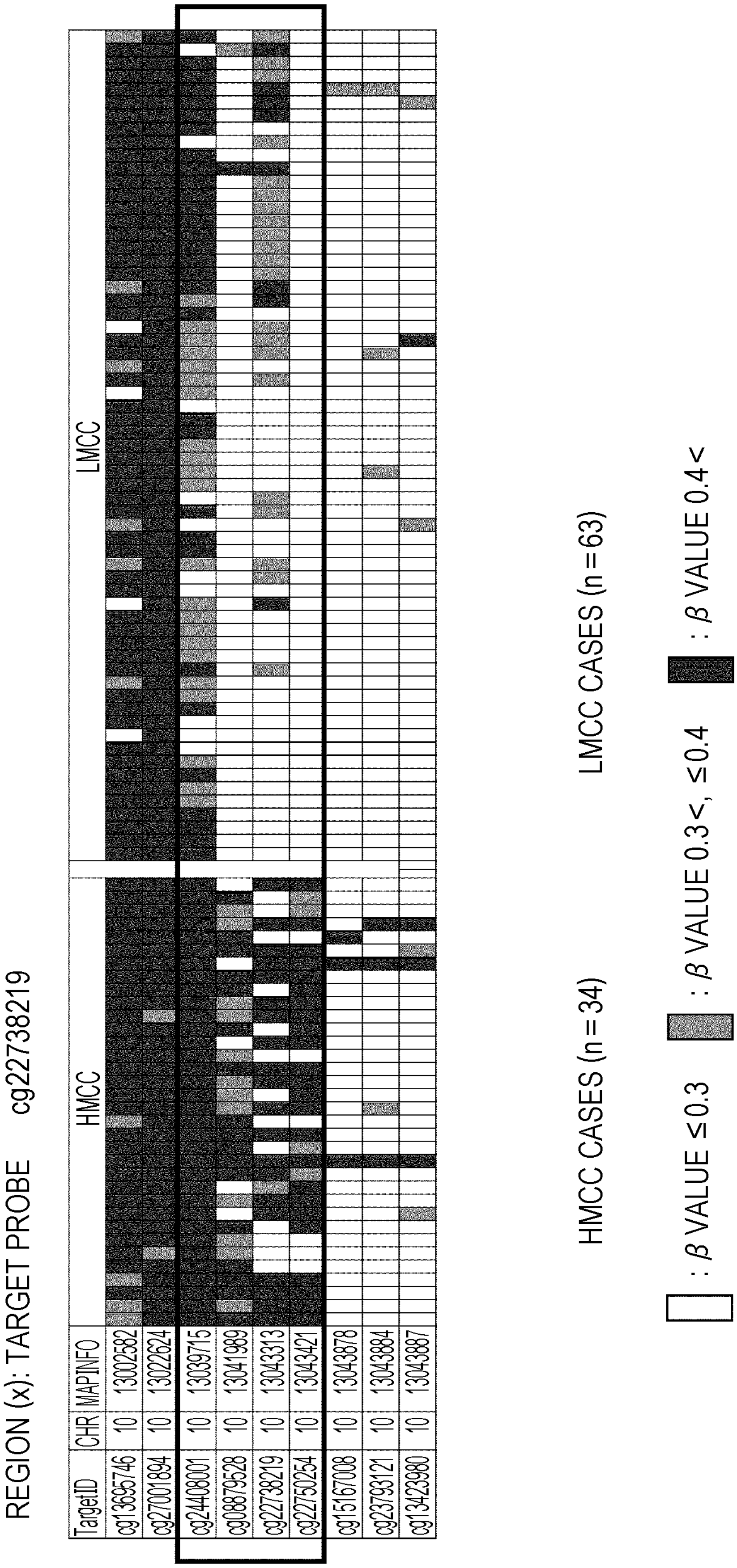
REGION (x): TARGET PROBE cg22738219
TargetID CHR MAPINFO
cg13695746 10 13002582
cg27001894 10 13022624
cg24408001 10 13039715
cg08879528 10 13041989
cg22738219 10 13043313
cg22750254 10 13043421
cg15167008 10 13043878
cg23793121 10 13043884
cg13423980 10 13043887
HMCC
LMCC
HMCC CASES (n=34)
LMCC CASES (n=63)
: β VALUE ≤0.3
: β VALUE 0.3<, ≤0.4
: β VALUE 0.4<

# FIG. 15

REGION (xi): TARGET PROBE cg20629468

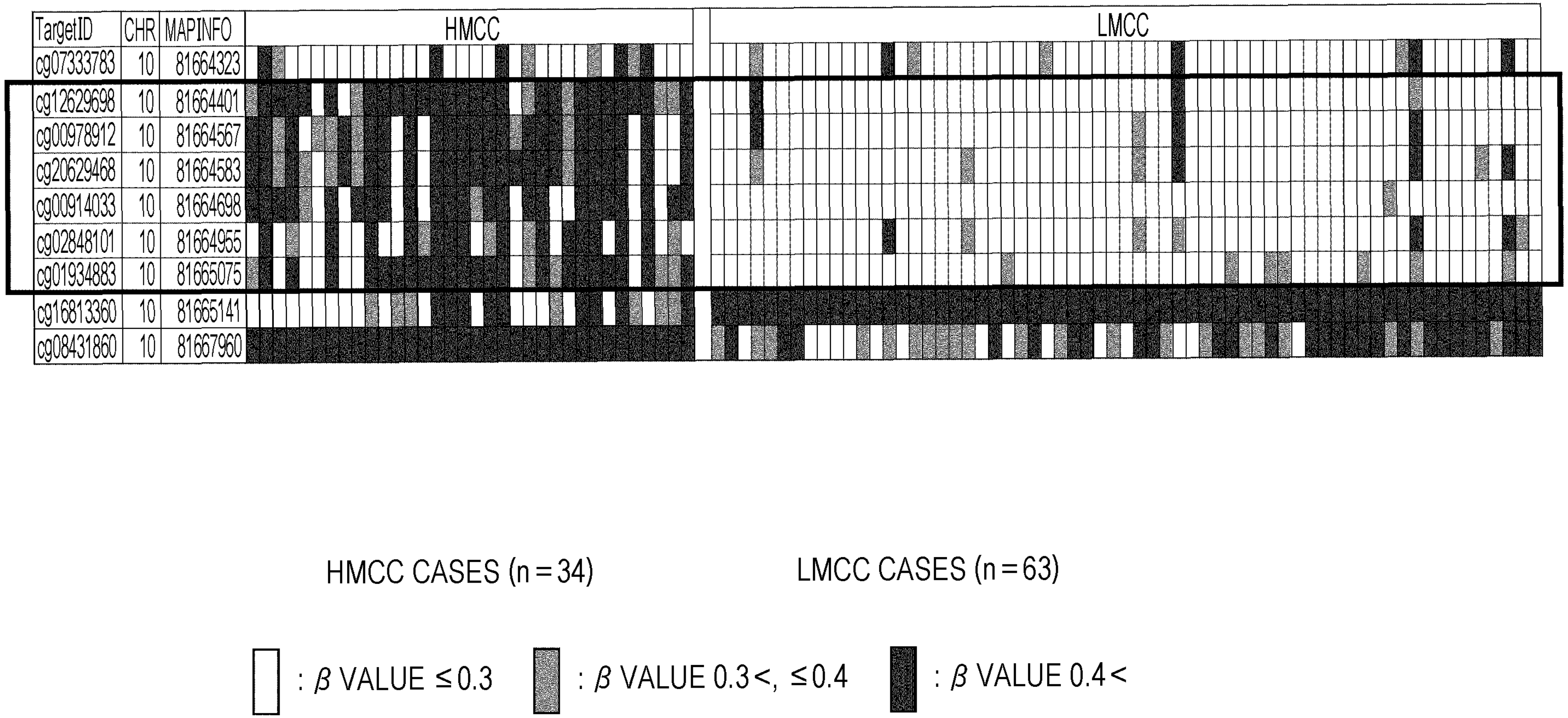

# FIG. 16

REGION (xii): TARGET PROBE cg20649951

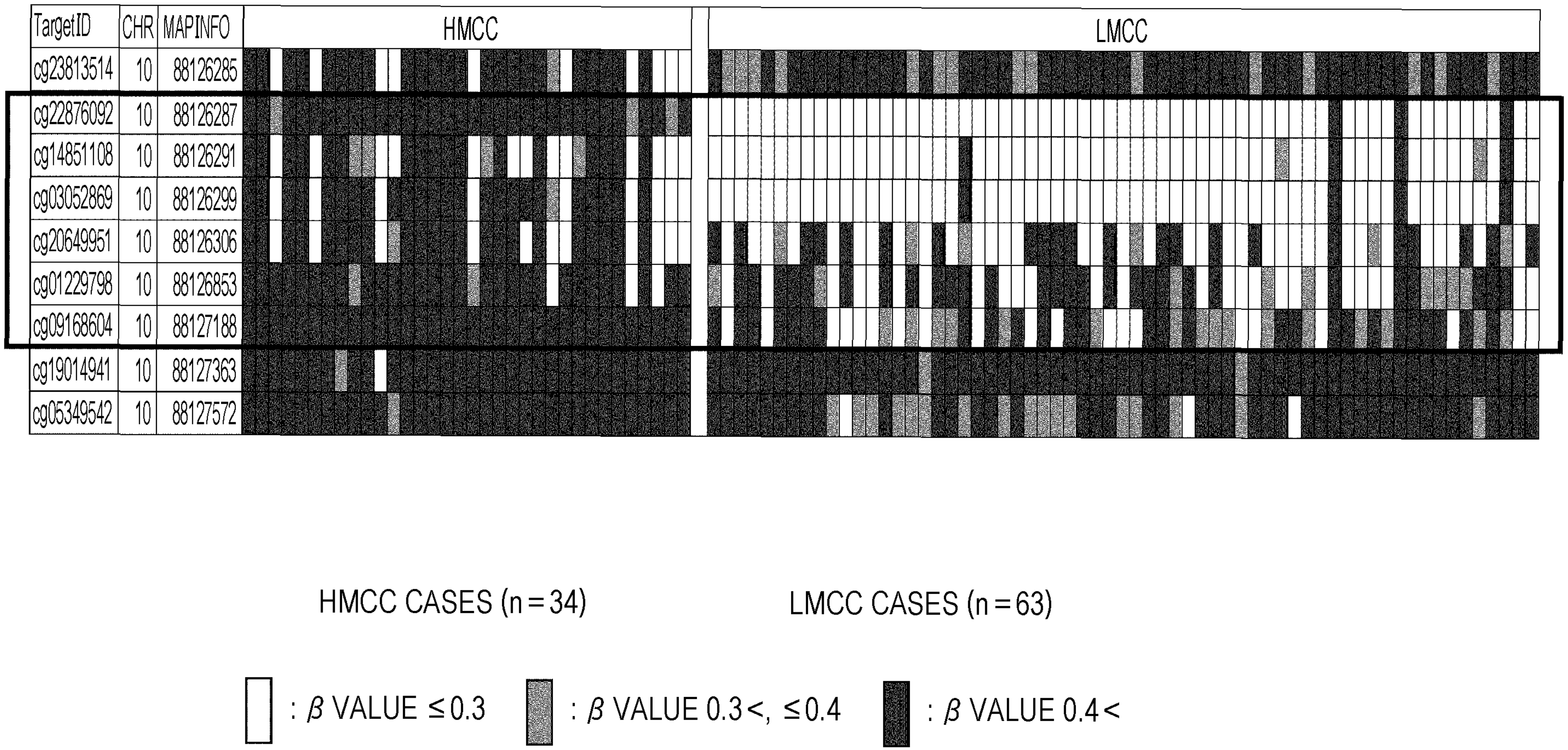

FIG. 17

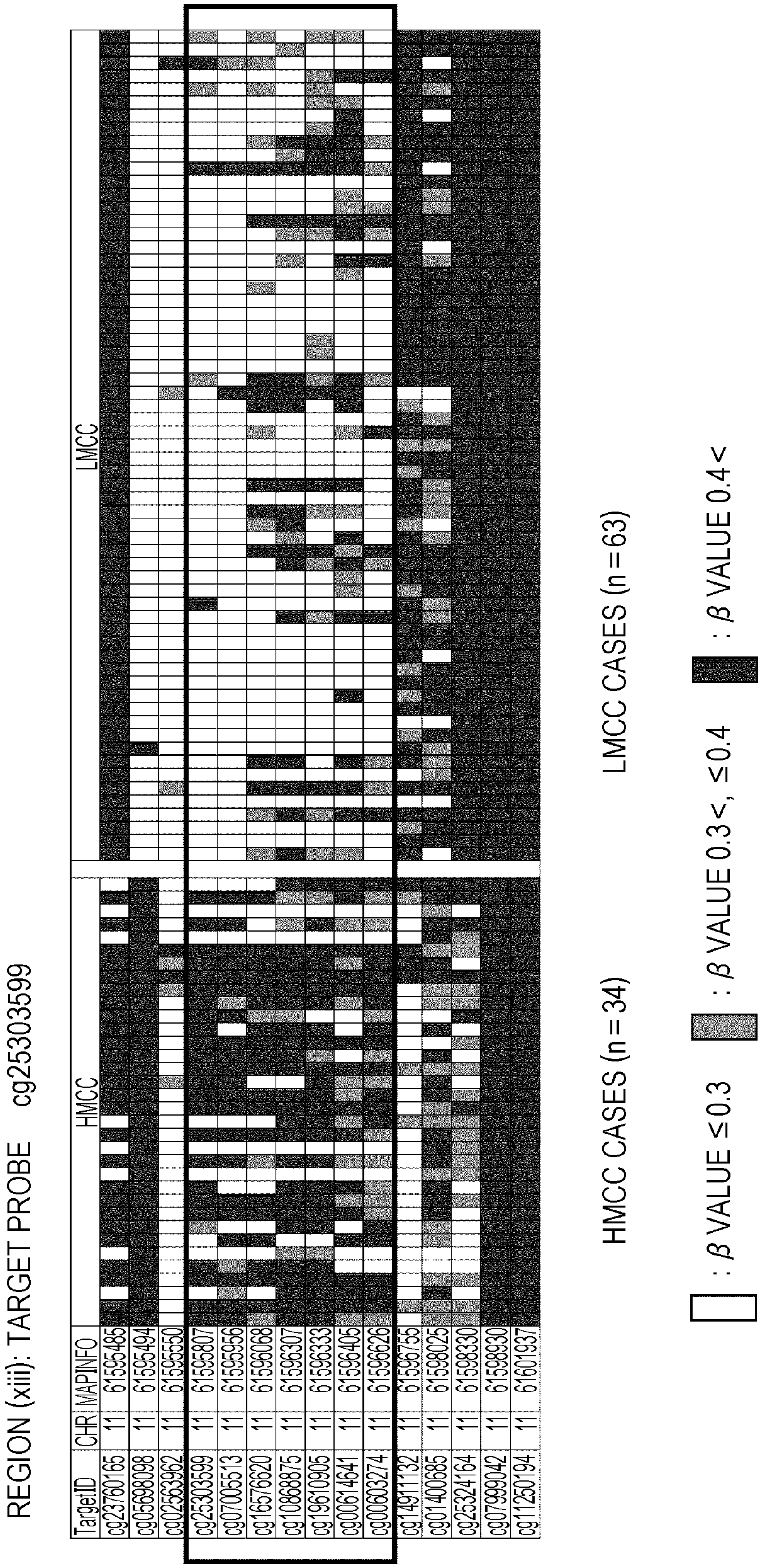
REGION (xiii): TARGET PROBE cg25303599
TargetID
CHR
MAPINFO
HMCC
LMCC
cg23760165 11 61595485
cg05698098 11 61595494
cg02563962 11 61595550
cg25303599 11 61595807
cg07005513 11 61595956
cg16576620 11 61596068
cg10868875 11 61596307
cg19610905 11 61596333
cg00614641 11 61596405
cg00603274 11 61596626
cg14911132 11 61596755
cg01400685 11 61598025
cg25324164 11 61598330
cg07999042 11 61598930
cg11250194 11 61601937
HMCC CASES (n = 34)
LMCC CASES (n = 63)
: β VALUE ≤0.3
: β VALUE 0.3<, ≤0.4
: β VALUE 0.4<

# FIG. 18

REGION (xiv): TARGET PROBE cg01557297

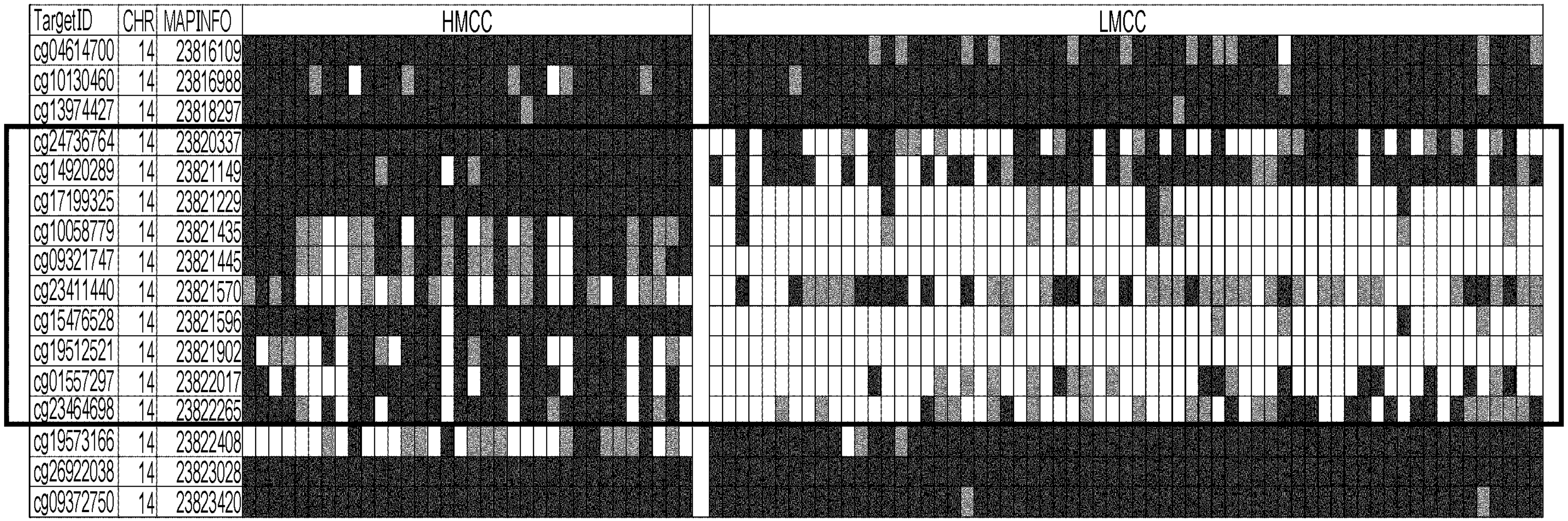


HMCC CASES (n = 34) LMCC CASES (n = 63)

□ : β VALUE ≤0.3 ▒ : β VALUE 0.3<, ≤0.4 ■ : β VALUE 0.4<

FIG. 19

REGION (xv): TARGET PROBE cg12379948

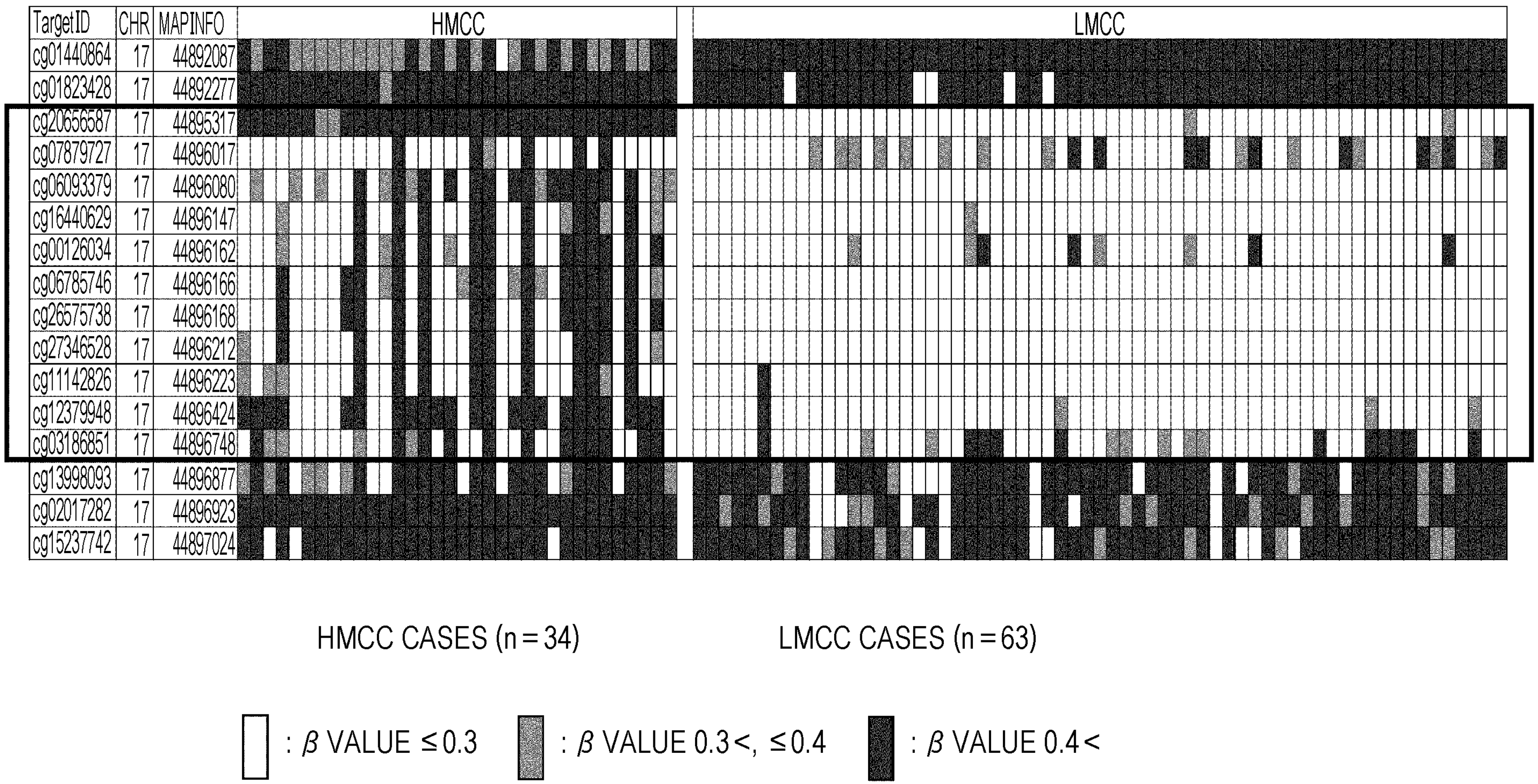

FIG. 20

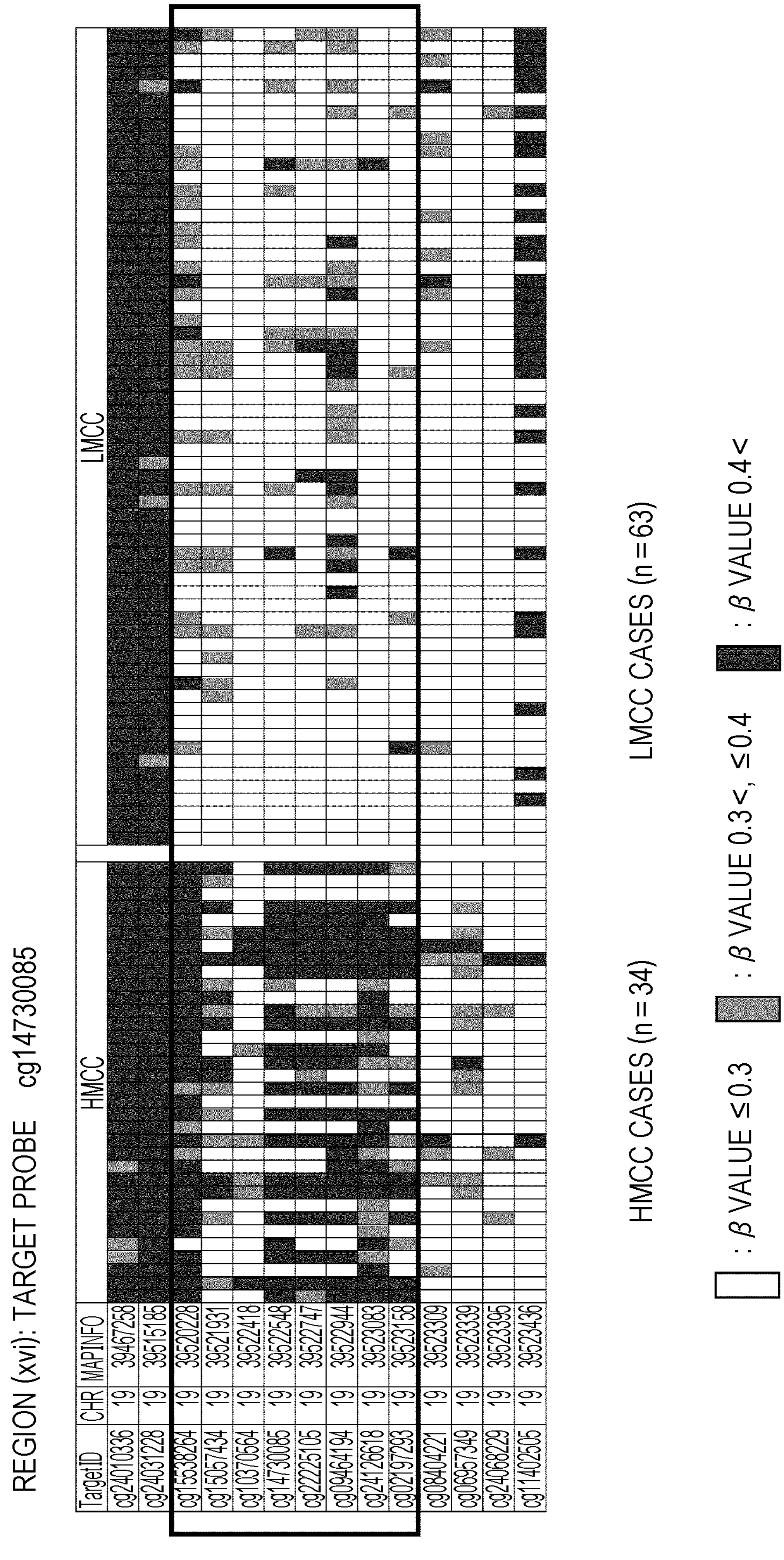
REGION (xvi): TARGET PROBE cg14730085
HMCC
LMCC
TargetID CHR MAPINFO
cg24010336 19 39467258
cg24031228 19 39515185
cg15538264 19 39520228
cg15057434 19 39521931
cg10370564 19 39522418
cg14730085 19 39522548
cg22225105 19 39522747
cg09464194 19 39522944
cg24126618 19 39523083
cg02197293 19 39523158
cg08404221 19 39523309
cg06957349 19 39523339
cg24068229 19 39523395
cg11402505 19 39523436
HMCC CASES (n=34)
LMCC CASES (n=63)
: β VALUE ≤0.3
: β VALUE 0.3<, ≤0.4
: β VALUE 0.4<

**INTERNATIONAL SEARCH REPORT**

International application No.
PCT/JP2020/021173

A. CLASSIFICATION OF SUBJECT MATTER
A61K 39/395(2006.01)n; A61P 1/04(2006.01)n; A61P 35/00(2006.01)n; C12Q 1/68(2018.01)i; C12Q 1/686(2018.01)i; C12Q 1/6869(2018.01)i; C12Q 1/6872(2018.01)i; C12Q 1/6886(2018.01)i; G01N 33/53 (2006.01)i
FI: C12Q1/68 ZNA; G01N33/53 M; C12Q1/686 Z; C12Q1/6886 Z; C12Q1/6872 Z; C12Q1/6869 Z; A61K39/395 T; A61P35/00; A61P1/04
According to International Patent Classification (IPC) or to both national classification and IPC

B. FIELDS SEARCHED
Minimum documentation searched (classification system followed by classification symbols)
A61K39/395; A61P1/04; A61P35/00; C12Q1/68; C12Q1/686; C12Q1/6869; C12Q1/6872; C12Q1/6886; G01N33/53

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | WO 2016/060278 A1 (TOHOKU UNIVERSITY) 21.04.2016 (2016-04-21) claims 2, 5, 10, 14, paragraph [0004], tables 7, 8 | 1-8, 10-16 |
| Y | paragraph [0043] | 9 |
| Y | WO 2013/033627 A1 (THE REGENTS OF THE UNIVERSITY OF CALIFORNIA) 07.03.2013 (2013-03-07) paragraph [0152] | 9 |
| A | OUCHI, K. et al., "DNA methylation status as a biomarker of anti-EGFR treatment for metastatic colorectal cancer", CANCER SCIENCE, December 2015, vol. 106, no. 12, pp. 1722-1729 entire text, all drawings | 1-16 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 July 2020 (28.07.2020) | 11 August 2020 (11.08.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/021173

C (Continuation). DOCUMENTS CONSIDERED TO BE RELEVANT

| Category* | Citation of document, with indication, where appropriate, of the relevant passages | Relevant to claim No. |
|---|---|---|
| A | KIM, J. C. et al., "Genome-wide identification of possible methylation markers chemosensitive to targeted regimens in colorectal cancers", J. CANCER RES. CLIN. ONCOL., 18 August 2011, vol. 137, pp. 1571-1580 entire text, all drawings | 1-16 |

Form PCT/ISA/210 (continuation of second sheet) (January 2015)

**INTERNATIONAL SEARCH REPORT**
Information on patent family members

International application No.
PCT/JP2020/021173

| Patent Documents referred in the Report | Publication Date | Patent Family | Publication Date |
|---|---|---|---|
| WO 2016/060278 A1 | 21 Apr. 2016 | US 2017/0356051 A1 claims 2, 5, 10, 14, paragraphs [0004], [0064], tables 7, 8 | |
| WO 2013/033627 A1 | 07 Mar. 2013 | US 2013/0129668 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019103299 A
- WO 2016060278 A1


### Non-patent literature cited in the description


- **SAMBROOK, J. et al.** Molecular Cloning: A Laboratory Manual. ColdSpringHarborLaboratory Press, 1998

SEQUENCE LISTING

<110> TOHOKU UNIVERSITY
RIKEN GENESIS CO., LTD.

<120> Method of testing sensitivity of drug treatment for colorectal cancer

<130> P20-076WO

<150> JP 2019-103299
<151> 2019-05-31

<160> 51

<170> PatentIn version 3.5

<210> 1
<211> 21
<212> DNA
<213> Artificial Sequence

<220>
<223> Primer

<400> 1
tagtattcga agtttcgttc g 21

<210> 2
<211> 24
<212> DNA
<213> Artificial Sequence

<220>
<223> Primer

<400> 2
gaacctaaaa atactaaaac gacg 24

<210> 3
<211> 19
<212> DNA
<213> Artificial Sequence

<220>
<223> Probe

<400> 3
aaacgaacgt acgccccgc 19

<210> 4
<211> 17
<212> DNA
<213> Artificial Sequence

<220>
<223> Primer

<400> 4
cggtcgtgta acgaagc 17

<210> 5
<211> 17
<212> DNA
<213> Artificial Sequence

<220>
<223> Primer

<400> 5
ccgaacgcga acaacta 17

<210> 6
<211> 27
<212> DNA
<213> Artificial Sequence

<220>
<223> Probe

<400> 6
caaacgaacg aaacgaaaac tacgacg 27

<210> 7
<211> 19
<212> DNA
<213> Artificial Sequence

<220>
<223> Primer

<400> 7
agtagaggag gtaggcgtc 19

<210> 8
<211> 19
<212> DNA
<213> Artificial Sequence

<220>
<223> Primer

<400> 8
cgacgaatac tcgactacg 19

<210> 9
<211> 19
<212> DNA
<213> Artificial Sequence

<220>
<223> Probe

<400> 9
ctcgaacccc gaaacgcac 19

<210> 10

<211> 22
<212> DNA
<213> Artificial Sequence

<220>
<223> Primer

<400> 10
gagtcgaggt cgttatttta gc 22

<210> 11
<211> 19
<212> DNA
<213> Artificial Sequence

<220>
<223> Primer

<400> 11
aataaacaac gcccaaacg 19

<210> 12
<211> 22
<212> DNA
<213> Artificial Sequence

<220>
<223> Probe

<400> 12
ccccgaactc aaacgtaaaa cg 22

<210> 13
<211> 18
<212> DNA
<213> Artificial Sequence

<220>
<223> Primer

<400> 13
ggggaaagtt tttatcgc 18

<210> 14
<211> 22
<212> DNA
<213> Artificial Sequence

<220>
<223> Primer

<400> 14
ctaataacgt tacccacaac cg 22

<210> 15
<211> 30
<212> DNA
<213> Artificial Sequence

<220>
<223>  Probe

<400>  15
cccgaaaaaa ctacgaaata aaactaaccg                                        30

<210>  16
<211>  22
<212>  DNA
<213>  Artificial Sequence

<220>
<223>  Primer

<400>  16
tcgattacga tttcgttatt tc                                        22

<210>  17
<211>  23
<212>  DNA
<213>  Artificial Sequence

<220>
<223>  Primer

<400>  17
atacgaatac aaatacgaaa ccg                                        23

<210>  18
<211>  24
<212>  DNA
<213>  Artificial Sequence

<220>
<223>  Probe

<400>  18
caactcgttt acaacgacgc aacc                                        24

<210>  19
<211>  21
<212>  DNA
<213>  Artificial Sequence

<220>
<223>  Primer

<400>  19
tttgtaagag gcgtaaagtg c                                        21

<210>  20
<211>  24
<212>  DNA
<213>  Artificial Sequence

<220>
<223>  Primer

<400> 20
ctatattccg aaacaactaa ctcg 24

<210> 21
<211> 23
<212> DNA
<213> Artificial Sequence

<220>
<223> Probe

<400> 21
catccactcc aaaatccgaa tcg 23

<210> 22
<211> 24
<212> DNA
<213> Artificial Sequence

<220>
<223> Primer

<400> 22
gtttttaat tttttggttt tcgc 24

<210> 23
<211> 19
<212> DNA
<213> Artificial Sequence

<220>
<223> Primer

<400> 23
cgaaccgcga aatatacga 19

<210> 24
<211> 20
<212> DNA
<213> Artificial Sequence

<220>
<223> Probe

<400> 24
aacgtcgaaa acgcaaacgc 20

<210> 25
<211> 22
<212> DNA
<213> Artificial Sequence

<220>
<223> Primer

<400> 25
gaaattgatg tcggagttgt ac 22

<210> 26
<211> 20
<212> DNA
<213> Artificial Sequence

<220>
<223> Primer

<400> 26
gacgcgaata aataaaaacg 20

<210> 27
<211> 20
<212> DNA
<213> Artificial Sequence

<220>
<223> Probe

<400> 27
tacgaacgcg ccgataacgc 20

<210> 28
<211> 21
<212> DNA
<213> Artificial Sequence

<220>
<223> Primer

<400> 28
attacggtgt gggagtggta c 21

<210> 29
<211> 22
<212> DNA
<213> Artificial Sequence

<220>
<223> Primer

<400> 29
cgaaatacta tacgaccaaa cg 22

<210> 30
<211> 27
<212> DNA
<213> Artificial Sequence

<220>
<223> Probe

<400> 30
caacatacta aaaatacccg ccgactc 27

<210> 31

<211> 26
<212> DNA
<213> Artificial Sequence

<220>
<223> Primer

<400> 31
agattcgtta atagttgagt gtaggc 26

<210> 32
<211> 19
<212> DNA
<213> Artificial Sequence

<220>
<223> Primer

<400> 32
ccgaaaacga aataaaacg 19

<210> 33
<211> 20
<212> DNA
<213> Artificial Sequence

<220>
<223> Probe

<400> 33
cacgcgaacc cgataaaacg 20

<210> 34
<211> 19
<212> DNA
<213> Artificial Sequence

<220>
<223> Primer

<400> 34
gtttttcggg agagtcgag 19

<210> 35
<211> 29
<212> DNA
<213> Artificial Sequence

<220>
<223> Primer

<400> 35
aactaaacta aactaaacta aaacgaacg 29

<210> 36
<211> 21
<212> DNA
<213> Artificial Sequence

<220>
<223>  Probe

<400>  36
caaaacgcaa aaacgaacgc g                                                 21

<210>  37
<211>  21
<212>  DNA
<213>  Artificial Sequence

<220>
<223>  Primer

<400>  37
atgttgtttg tcgattgtga c                                                 21

<210>  38
<211>  22
<212>  DNA
<213>  Artificial Sequence

<220>
<223>  Primer

<400>  38
aaaaacaacc gtctatacaa cg                                                22

<210>  39
<211>  19
<212>  DNA
<213>  Artificial Sequence

<220>
<223>  Probe

<400>  39
aaccgacgcg aaaaaaccg                                                    19

<210>  40
<211>  19
<212>  DNA
<213>  Artificial Sequence

<220>
<223>  Primer

<400>  40
agattaagga ggcgttcgc                                                    19

<210>  41
<211>  23
<212>  DNA
<213>  Artificial Sequence

<220>
<223>  Primer

<400> 41
ctatcctcta actcaattac gcg 23

<210> 42
<211> 27
<212> DNA
<213> Artificial Sequence

<220>
<223> Probe

<400> 42
caactcgaaa aaatcccgct ctacaac 27

<210> 43
<211> 26
<212> DNA
<213> Artificial Sequence

<220>
<223> Primer

<400> 43
ggttttcgtt agtaaaggag ttattc 26

<210> 44
<211> 20
<212> DNA
<213> Artificial Sequence

<220>
<223> Primer

<400> 44
aaaaacgcga taaaccgaac 20

<210> 45
<211> 27
<212> DNA
<213> Artificial Sequence

<220>
<223> Probe

<400> 45
ctttaacccg aacgttcaca taacgaa 27

<210> 46
<211> 26
<212> DNA
<213> Artificial Sequence

<220>
<223> Primer

<400> 46
gtagttgtta gtttttcgtt cgtaac 26

<210> 47
<211> 19
<212> DNA
<213> Artificial Sequence

<220>
<223> Primer

<400> 47
gaaatcccac cttaaccgc 19

<210> 48
<211> 24
<212> DNA
<213> Artificial Sequence

<220>
<223> Probe

<400> 48
ttacgtccga taaatctacg cgcg 24

<210> 49
<211> 23
<212> DNA
<213> Artificial Sequence

<220>
<223> Primer

<400> 49
ggaggtaggg agtatatagg ttg 23

<210> 50
<211> 25
<212> DNA
<213> Artificial Sequence

<220>
<223> Primer

<400> 50
actcctccct taaaaattac aaaaa 25

<210> 51
<211> 23
<212> DNA
<213> Artificial Sequence

<220>
<223> Probe

<400> 51
ccaccaccca acacacaata aca 23